# EUROPEAN PATENT SPECIFICATION

(11) **EP 2 841 088 B1**
(45) Date of publication and mention of the grant of the patent: **17.06.2020**
(21) Application number: 13768168.0
(22) Date of filing: 28.03.2013
(51) Int. Cl.: A61K 38/22, A61K 45/06, A61P 31/00, A61P 31/04

(54) **USE OF THYMOSIN ALPHA FOR THE TREATMENT OF SEPSIS**
VERWENDUNG VON THYMOSIN ALPHA ZUR BEHANDLUNG VON SEPSIS
UTILISATION DE THYMOSINE ALPHA POUR LE TRAITEMENT DE LA SEPTICÉMIE

(30) Priority: 30.03.2012 US 201261618563 P; 07.05.2012 US 201261643824 P; 15.03.2013 US 201313835107
(43) Date of publication of application: 04.03.2015
(73) Proprietor: SciClone Pharmaceuticals International Ltd., Grand Cayman, KY1-1108 (KY); First Affiliated Hospital, Sun Yat-Sen University, Guangzhou 510080 (CN)
(72) Inventor: GUAN, Xiangdong, Guangzhou 510080 (CN); WU, Jianfeng, Guangzhou 510080 (CN); TUTHILL, Cynthia, Menlo Park, California 94025 (US)
(74) Representative: Cooley (UK) LLP
(86) International application number: PCT/US2013/034394
(87) International publication number: WO 2013/149030

(56) References cited:
- WO-A1-95/09647
- DATABASE MEDLINE [Online] US NATIONAL LIBRARY OF MEDICINE (NLM), BETHESDA, MD, US; January 2009 (2009-01), LI YUMIN ET AL: "A new immunomodulatory therapy for severe sepsis: Ulinastatin Plus Thymosin {alpha} 1.", XP002743765, Database accession no. NLM19033321 & JOURNAL OF INTENSIVE CARE MEDICINE 2009 JAN-FEB, vol. 24, no. 1, January 2009 (2009-01), pages 47-53, ISSN: 0885-0666
- DATABASE MEDLINE [Online] US NATIONAL LIBRARY OF MEDICINE (NLM), BETHESDA, MD, US; March 2009 (2009-03), SU LEI ET AL: "[Clinical effects of ulinastatin and thymosin alpha1 on immune-modulation in septic patients].", XP002743766, Database accession no. NLM19278583 & ZHONGGUO WEI ZHONG BING JI JIU YI XUE = CHINESE CRITICAL CARE MEDICINE = ZHONGGUO WEIZHONGBING JIJIUYIXUE MAR 2009, vol. 21, no. 3, March 2009 (2009-03), pages 147-150, ISSN: 1003-0603
- DATABASE MEDLINE [Online] US NATIONAL LIBRARY OF MEDICINE (NLM), BETHESDA, MD, US; January 2009 (2009-01), YU YANG ET AL: "[Evaluation of efficacy of thymosin alpha1 in the treatment of sepsis: a systematic review].", XP002743767, Database accession no. NLM19141185 & ZHONGGUO WEI ZHONG BING JI JIU YI XUE = CHINESE CRITICAL CARE MEDICINE = ZHONGGUO WEIZHONGBING JIJIUYIXUE JAN 2009, vol. 21, no. 1, January 2009 (2009-01), pages 21-24, ISSN: 1003-0603
- YING ZHANG ET AL: "Thymosin [alpha] 1 - and Ulinastatin-Based Immunomodulatory Strategy for Sepsis Arising from Intra-Abdominal Infection Due to Carbapenem-Resistant Bacteria", THE JOURNAL OF INFECTIOUS DISEASES, vol. 198, no. 5, 1 September 2008 (2008-09-01), pages 723-730, XP055164137, ISSN: 0022-1899, DOI: 10.1086/590500

## Description

### CROSS-REFERENCE TO RELATED APPLICATIONS

### FIELD OF THE INVENTION

The present invention relates to the field of sepsis, including an alpha thymosin peptide regimen for use in the prevention of, reduction in severity, or treatment of sepsis.

### BACKGROUND

Septic shock is a condition in which infection is widely disseminated in many areas of the body, the infection generally being disseminated through the blood from one tissue to another and causing extensive damage. Septic shock can occur with numerous medical conditions, including (1) peritonitis caused by the spread of infection from the uterus and fallopian tubes; (2) peritonitis resulting from rupture of the gut, sometimes caused by intestinal disease or wounds; (3) generalized infection resulting from spread of a simple infection; (4) generalized gangrenous infection resulting specifically from gas gangrene bacilli; and (5) infection spreading into the blood from the kidney, urinary tract or the abdomen.

Sepsis frequently occurs as a hospital-acquired infection, contributing to the significant patient mortality and morbidity, and add significantly to the overall cost of healthcare [Michael Klompas, Prevention of ventilator-associated pneumonia, Expert Rev. Anti Infect. Ther. 8(7), 791-800 (2010); Wheeler DS et al., Novel Pharmacologic Approaches to the Management of Sepsis: Targeting the Host Inflammatory Responses, Recent Pat. Inflamm. Allergy Drug Discov. 3(2):96-112 (2009)]. In fact, sepsis was reported as the 10^{th} leading cause of death in 2004. (See, e.g., Wheeler et al. (2009)). In fact, 750,000 people annually in the United States are diagnosed with severe sepsis and of those, 215,000 will die from the severe sepsis. (See, Angus DC, et al., Epidemiology of severe sepsis in the United States: analysis of incidence, outcome, and associated costs of care. Crit Care Med., 29:1303-1310 (2001)).

WO95/09647 A1 describes the use of thymosin-alpha in the treatment of sepsis in mammals by parenteral administration. However, the experiments in this disclosure relate only to mouse and rat models of sepsis shock. It was known that sepsis treatments which obtained promising results in animal models often subsequently failed to demonstrate clinical efficacy in human trials (see Bredan and Cauwels review, Libyan J. Med, AOP:071017, pp.34-36, 2008, Rittirsch, J. of Leukocyte Biology, 81, pp.137-143, 2007, and Ulloa et al. Curr Pharm Des. 15:16, pp.1918-1935, 2009).

Li et al. J. of Intensive Care Medicine, 24:1, pp.47-53, 2009, and Su et al. Chinese Critical Care Medicine, 21:3, pp.147-150, 2009 describe the combined use of alpha thymosin peptide and ulinastatin in the treatment of sepsis. The combination of alpha thymosin peptide and ulinastatin for the treatment of sepsis due to carbapenem-resistant bacteria is described in Ying Zhang et al. J. of Infectious Diseases, 198:5, pp.723-730, 2008.

Yu Yang et al. Chinese Critical Care Medicine, 21:1, pp.21-24, 2009, describes the use of alpha thymosin peptide in the treatment of sepsis. This reference concludes that thymosin alpha 1 improves the immune-suppression and helps recovery of the patient from disease. However, not enough evidence is found to show that alpha thymosin peptide decreases mortality rate.

A strong and rapid immune response to pathogens is important for preventing, treating and/or reducing the severity of sepsis due to viral, bacterial, and fungal infections. A means for reducing the impact of infection and to help to prevent, reduce or treat sepsis is of great need.

### SUMMARY OF THE INVENTION

The invention is defined by the appended claims.

The present invention provides alpha thymosin for use in a method for treating sepsis in a human, the method comprising administering a regimen of alpha thymosin peptide to the human, wherein the administration does not contain a protease inhibitor, and wherein the treatment reduces mortality.

In some embodiments, the subject is immune deficient.

In some embodiments, the sepsis is hospital acquired. In some embodiments, the sepsis is due to bacterial, fungal or viral infection.

In some embodiments, the subject shows one or more signs or symptoms of an infection. In some embodiments, the subject shows one or more signs or symptoms of sepsis.

In some embodiments, the alpha thymosin peptide is administered at least about 0.5 mg per day, or at about 0.5 to about 3 mg per day, or at about 1.6 to about 3.2 mg per day or at least at about 1.6 mg per day.

In some embodiments, the alpha thymosin peptide is administered intravenously. In some embodiments, the alpha thymosin peptide is administered by continuous infusion or subcutaneous injection.

In some embodiments, alpha thymosin peptide is administered from about 1 to 4 times daily. In some embodiments, alpha thymosin peptide is administered approximately twice daily. In some embodiments, alpha thymosin peptide is administered approximately once per day. In some embodiments, alpha thymosin peptide is administered about twice per day for at least 5 days (e.g., from 5 to 14 days). In some embodiments, alpha thymosin peptide is administered about twice per day for about 5 to 10 days (or about 5 days) followed by about once per day for at least two days, or about 2 to 7 days, or about 2 days.

In some embodiments, the patient has been diagnosed as having sepsis. The sepsis may be of bacterial, viral, fungal, or mixed or unknown etiology.

In some embodiments, the sepsis is associated with an infectious organism selected from *Lysteria monocytogenes, Pseudomonas sp. (e.g., P. aeruginosa), Serratia marcescens, Clostridium difficile, Staphylococcus aureus, Staphylococcus sp., Acinetobacter spp., Enterococcus sp., Enterobacter sp., E. coli, Klebsiella sp., Streptococcus (e.g., S. pneumoniae), Haemophilus influenzae,* and *Neisseria meningitidis.*

In some embodiments, the sepsis is associated with one or more drug resistant microorganisms, such as *Staphylococcus aureus, Staphylococcus sp., Enterococcus sp., Pseudomonas sp., Klebsiella sp., E. coli,* and/or *Clostridium Difficile.* In some embodiments, the sepsis is associated with a methicillin-resistant or vancomycin-resistant *Staphylococcus aureus,* including intermediate resistant isolates, and/or carbapenum-resistant *E*. *coli, Klebsiella,* or *Pseudomonas,* including intermediate resistant isolates.

The alpha thymosin peptide regimen may be administered concurrently with the standard of care, such as antibiotic or antiviral therapy.

Other objects and aspects of the invention will be apparent from the following detailed description.

### DESCRIPTION OF THE FIGURES

**Figure 1** provides a diagram of the Study Profile for the study described in Example 1. (Tα1, thymosin alpha 1)
**Figure 2** describes Kaplan-Meier estimate of the probability of 28-day survival in the presence and absence of thymosin alpha 1. (Tα1, thymosin alpha 1)
**Figure 3** describes the analysis of the rates and risks of death from any cause within 28 days in prespecified subgroups. APACHE, Acute Physiology and Chronic Health Evaluation; CI, confidence interval; HLA-DR, human leukocyte antigen-DR; SOFA, Sequential Organ Failure Assessment. (Tα1, thymosin alpha 1)

### DETAILED DESCRIPTION OF THE INVENTION

The present invention is based in part on the discovery that certain regimen of alpha thymosin peptide therapy can be used to treat sepsis, especially certain low dosage administration of alpha thymosin peptide therapy can provide statistically significant therapeutic effect for the treatment of sepsis. Accordingly the present invention provides alpha thymosin for use in a method for treating sepsis in a human, the method comprising administering a regimen of alpha thymosin peptide to the human, wherein the administration does not contain a protease inhibitor, and wherein the treatment reduces mortality.

According to the present invention, sepsis includes any recognized form of sepsis, e.g., hospital-acquired sepsis, medical procedure related sepsis, medical device related sepsis, severe sepsis, or septic shock. Sepsis also includes any recognized condition or symptom associated with sepsis. In general, symptoms of sepsis include but are not limited to fever above 101.3°F (38.5°C) or below 95°F (35°C), heart rate higher than 90 beats per minute, respiratory rate higher than 20 breaths a minute, and probable or confirmed infection (*i.e*., presence of one or more infectious agents such as bacteria, fungi or viruses). Typically, a clinical diagnosis of sepsis includes the presence of a least two symptoms selected from the sepsis symptoms. Symptoms of severe sepsis include but are not limited to significantly decreased urine output, abrupt change in mental status, decrease in platelet count, difficulty breathing, abnormal heart pumping function and abdominal pain. Typically, a clinical diagnosis of severe sepsis includes the presence of a least one additional symptom selected from the severe sepsis symptoms, the presence of which is indicative of organ failure. Symptoms of septic shock can include but are not limited to extremely low blood pressure that does not respond to simple fluid replacement. Typically, a clinical diagnosis of septic shock includes the presence of at least one additional symptom selected from the septic shock symptoms.

In general, sepsis can be caused due to a variety of infectious agents, including bacteria, fungi, viruses and parasites, and can proceed from merely infection to multiple organ dysfunction syndrome (MODS) and eventual death if untreated. In some embodiments, sepsis may involve, for example, bacteremia or fungal infection, such as candidemia or aspergillis infection. In some embodiments, sepsis may result from severe injury, severe wound, or burn, and may be a post-surgical infection

According to the present invention, treatment of sepsis includes any form of treating or preventing sepsis, e.g., reducing any symptom of sepsis, reducing the severity of any symptom of sepsis, delaying the onset of sepsis, shortening the duration of one or more symptoms of sepsis, reducing the opportunity or occurrence of sepsis, treating or inhibiting any cause or condition associated with sepsis, reducing any clinical criteria or measurement of the degree or condition of sepsis, e.g., ICU frequency, ICU stay, ICU free days, duration of ventilation, ventilation free days, mortality, e.g., 28 day mortality, in-ICU mortality, in-hospital mortality, etc., dynamic change of SOFA, HLA-DR expression, etc.

In one embodiment, the invention involves administering a regimen of alpha thymosin peptide to enhance immune responses to pathogen exposure, or potential pathogen exposure in order to treat sepsis.

Thymosin alpha was originally isolated from bovine thymus, where it was shown to reconstitute "immune function" in thymectomized animal models. Thymosin is thought to play a role in inflammatory and innate immune responses, and to facilitate discrimination of self from non-self in mammals. Activation of certain Toll-like receptors (TLR; also known as PAMP or pathogen-associated molecular patterns) by thymosin leads to stimulation of intracellular signal transduction pathways resulting in expression of co-stimulatory molecules, pro-inflammatory cytokines, nitric oxide, and eicosanoids. Thymosin may affect, for example, precursor cells, dendritic cells, T cells, B cells, and NK cells.

Without intending to be bound by theory, it is believed that alpha thymosin peptides (e.g., TA1), among other things, activate Toll-like Receptor 9 (TLR), resulting in increases in Th1 cells, B cells, and NK cells, thereby priming the immune system for an enhanced immune response. For example, TA1 may increase or enhance lymphocytic infiltration, secretion of chemotactic cytokines, maturation and differentiation of dendritic cells, secretion of thymopoeitic cytokines including IFN-α, IL-7, and IL-15, and B cell production of antibodies.

According to the present invention, alpha thymosin peptides to be used in methods of the present invention include thymosin alpha 1 ("TA1"; "Tα1"), and peptides having structural homology to TA1. TA1 is a peptide having the amino acid sequence (N-acetyl)-Ser-Asp-Ala-Ala-Val-Asp-Thr-Ser-Ser-Glu-Ile-Thr-Thr-Lys-Asp-Leu-Lys-Glu-Lys-Lys-Glu-Val-Val-Glu-Glu-Ala-Glu-Asn-OH (SEQ ID NO: 1). The amino acid sequence of TA1 is disclosed in U.S. Patent 4,079,127. TA1 is a non-glycosylated 28-amino acid peptide having an acetylated N-terminus, and a molecular weight of about 3108. A synthetic version of TA1 is commercially available in certain countries under the trade name ZADAXIN.

In some embodiments, alpha thymosin peptides suitable for use in methods of the present invention include naturally occurring TA1 (e.g., TA1 purified or isolated from tissues), synthetic TA1, recombinant TA1 as well as any suitable TA1 analogs with substantially the same or better function of TA1. In some other embodiments, the thymosin peptide comprises the amino acid sequence of SEQ ID NO:1 (where an acylated, e.g., acetylated, N-terminus is optional). In some embodiments, the thymosin peptide comprises an amino acid sequence that is substantially similar to TA1, and maintains the immunomodulatory activity of TA1. The substantially similar sequence may have, for example, from about 1 to about 10 amino acid deletions, insertions, and/or substitutions (collectively) with respect to TA1. For example, the thymosin peptide may have from about 1 to about 5 (e.g., 1, 2, or 3) amino acid insertions, deletions, and/or substitutions (collectively) with respect to TA1.

In some embodiments, the alpha thymosin peptide may comprise an abbreviated TA1 sequence, for example, having deletions of from 1 to about 10 amino acids, or from about 1 to 5 amino acids, or 1, 2 or 3 amino acids with respect to TA1. Such deletions may be at the N- or C-terminus, and/or internal, so long as the immunomodulatory activity of the peptide is substantially maintained. Alternatively, or in addition, the substantially similar sequence may have from about 1 to about 5 amino acid insertions (e.g., 1, 2, or 3 amino acid insertions) with respect to TA1, where the immunomodulatory activity of TA1 is substantially maintained. Alternatively, or in addition, the substantially similar sequence may have from 1 to about 10 amino acid substitutions, where the immunomodulatory activity is substantially maintained. For example, the substantially similar sequence may have from 1 to about 5, or 1, 2, or 3 amino acid substitutions, which may include conservative and non-conservative substitutions. In some embodiments, the substitutions are conservative. Generally, conservative substitutions include substitutions of a chemically similar amino acid (e.g., polar, non-polar, or charged). Substituted amino acids may be selected from the standard 20 amino acids or may be a non-standard amino acid (*e.g*., a conserved non-standard amino acid).

In some embodiments, the alpha thymosin peptide includes a TA1 sequence substituted with one or more non-natural or modified amino acids. In some other embodiments, the alpha thymosin peptide includes a TA1 sequence conjugated to one or more entities. In some embodiments, the alpha thymosin peptide is pegylated to increase its half-life in circulation. Such strategies for increasing the half-life of therapeutic proteins are well known.

In some embodiments, the thymosin peptide comprises an amino acid sequence having at least 70% sequence identity to SEQ ID NO:1, while maintaining the immunomodulatory activity of TA1. For example, the thymosin peptide may comprise an amino acid sequence having at least 80%, 85%, 90%, 91%, 92%, 93%, 94%, 95%, 96% or 97% sequence identity to SEQ ID NO:1. The thymosin peptide may comprise an amino acid sequence having 100% sequence identity to SEQ ID NO:1. In all cases, the N-terminus may be optionally acylated (e.g., acetylated) or alkylated, for example, with a C1-C10 or C1-C7 acyl or alkyl group.

In certain embodiments, the substantially similar and homologous peptides described above may function at a level of at least about 50%, 60%, 70%, 75%, 80%, 85%, 90%, 91%, 92%, 93%, 94%, 95%, 96% or 97% relative to TA1 (SEQ ID NO:1).

In general, the alpha thymosin peptide may be prepared synthetically, for example, by solid phase synthesis, or may be made recombinantly and purified by known techniques.

In some embodiments, the alpha thymosin peptide may be provided in lyophilized form, and reconstituted with sterile (e.g., aqueous) diluent prior to administration.

According to the present invention, the alpha thymosin peptide of the present invention is administered in a regimen for the treatment of sepsis. Such regimen includes dosage per administration, per day, as well as number of days per treatment cycle, or combinations thereof.

In general, the alpha thymosin can be administered at a dosage of from about 0.2 mg to 20 mg, 0.2 mg to 15 mg, 0.4 to 10 mg, 0.5 mg to 8 mg. 0.5 mg to 6 mg, 0.5 mg to 3 mg. In some embodiments, the alpha thymosin is administered at 0.2 mg, 0.5mg, 0.4 mg, 0.8 mg, 1 mg, 1.6 mg, 3 mg, 3.2 mg, 6.4 mg or about 8 mg. In some embodiments the alpha thymosin peptide is administered to a human patient at a dose corresponding to at least about 0.5 mg (*e.g*., at least about 0.8 mg, or at least about 1.6 mg), at least about 3 mg (*e.g*., at least about 3.2 mg), or at least about 5 mg (*e.g*., at least about 6.4 mg) of TA1. In some embodiments, the thymosin peptide is administered within the range corresponding to about 0.1 to 20 mg of TA1, or about 1 to 10 mg of TA1, or about 2 to 10 mg of TA1, or about 2 to 8 mg of TA1, or about 2 to 7 mg of TA1. In certain embodiments, the dosage unit is within a range of about 3 to 6.5 mg, such as about 3.2 or 6.4 mg of TA1. In certain embodiments, the TA1 dose is adjusted to the size of the patient, and may be provided at from 10 to 100 µg / kg (*e.g*., about 20, 40, 60, or 80 µg / kg). Dosages may also be adjusted for the condition of each patient as well as other drugs taken by the patient. In addition, dosages may be adjusted according to the species of the subject, but in each case, approximately correspond to the human equivalent of TA1 (mg/kg).

In some embodiments, such dosage is administered hourly, daily, weekly or monthly.

In some embodiments alpha thymosin peptide is administered hourly, about every 1 to 24 hours, 1 to 20 hours, 1 to 16 hours, 1 to 12 hours, 1 to 8 hours, 1 to 6 hours, 1 to 4 hours, 1 to 2 hours or every hour. In some embodiments, the alpha thymosin peptide is administered about every 2, 3, 5, 5, or 6 hours, or is administered about every 10 minutes, 15 minutes, 30 minutes, 45 minutes or 60 minutes.

Alternatively, the thymosin peptide can be administered by a plurality of injections (sub-doses of thymosin peptide) on a treatment day, so as to substantially continuously maintain an immune stimulating-effective amount of the thymosin peptide in the patient's circulatory system for a longer period of time. Suitable injection regimens may include an injection every 2, 3, 4, 6, etc. hours on the day of administration (*e.g.*, from 2 to 5 injections), so as to substantially continuously maintain the immune stimulating-effective amount of the thymosin peptide in the patient's circulatory system on the day of thymosin treatment.

In some embodiments, the TA1 may be administered by continuous infusion. Continuous infusion of TA1 is described in detail in US 2005/0049191. Briefly, continuous infusion of thymosin peptide maintains an immune stimulating-effective amount of a thymosin peptide in a patient's circulatory system for a longer period. In some embodiments, the thymosin peptide may be administered to the patient for treatment periods of at least about 2, 4, 6, 10, 12 hours, or longer, which may improve effectiveness in some embodiments. The infusion may be carried out by any suitable means, such as by minipump.

In some embodiments, the alpha thymosin is administered by continuous infusion for about 1 to 168 hours, 1 to 144 hours, 1 to 120 hours, 1 to 96 hours, 1 to 72 hours, 1 to 48 hours, 1 to 24 hours, 1 to 20 hours, 1 to 16 hours, 1 to 12 hours 1 to 10 hours, 1 to 8 hours, 1 to 6 hours, 1 to 4 hours to 1 to 2 hours. In some embodiments, the alpha thymosin peptide is administered by continues infusions for about 10 minutes, 15 minutes, 30 minutes, 45 minutes or 60 minutes. In some embodiments, the alpha thymosin is administered by continuous infusion for about 1 hour, 2 hours, 3 hours, 4 hours, 5 hours, 6 hours, 7 hours, 8 hours, 10 hours, 12 hours, 24 hours or more. In some embodiments, the continuous infusion periods are separated by periods of non-infusion (*i.e*., periods where no alpha thymosin is administered). In some embodiments, the non-infusion period ranges from 1 to 168 hours, 1 to 144 hours, 1 to 120 hours, 1 to 96 hours, 1 to 72 hours, 1 to 48 hours, 1 to 24 hours, 1 to 20 hours, 1 to 16 hours, 1 to 12 hours 1 to 10 hours, 1 to 8 hours, 1 to 6 hours, 1 to 5 hours, 1 to 4 hours, 1 to 3 hours, 1 to 2 hours. In some embodiments, the non-infusion period is about 1 hour, 2 hours, 3 hours, 4 hours, 5 hours, 6 hours, 7 hours, 8 hours, 10 hours, 12 hours, 24 hours or more.

In some embodiments, a predetermined amount of alpha thymosin peptides, e.g., immune stimulating-effective amount of a thymosin peptide (*e.g*. TA1) may be substantially continuously maintained in a patient's circulatory system by administering the TA1 peptide to the patient at a rate within a range of about 0.0001-0.1 mg/hr/Kg patient body weight. Exemplary administration rates are within a range of about 0.0003-0.03 mg/hr/Kg patient body weight. For continuous infusion, the TA1 peptide is present in a pharmaceutically acceptable liquid carrier, such as water for injection, or saline in physiological concentrations.

In some embodiments, the thymosin is administered about every 1 to 20 days, every 1 to 15 days, every 1 to 10 days, every 1 to 7 days, every 1 to 5 days, every 1 to 3 days or daily. In some embodiments, the alpha thymosin is administered for about 1 to 100 days, 1 to 90 days, 1 to 80 days, 1 to 70 days, 1 to 50 days, 1 to 40 days, 1 to 30 days, 1 to 20 days, 1 to 15 days, 1 to 10 days, 1 to 7 days, 1 to 5 days, 1 to 3 days, 1 to 14 days, 5 to 14 days or 1 to 2 days. In some embodiments, the alpha thymosin is administered for about 1, 2, 3, 4, 5, 6, 7, 8, 9, 10, 11, 12, 13, 14, 15, 16, 17, 18 ,19 , 20, 25, 30 or more days. In some embodiments, alpha thymosin peptide is administered about twice per day for at least 5 days(e.g., from 5 to 14 days). In some embodiments, alpha thymosin peptide is administered about twice per day for about 5 to 10 days (or about 5 days) followed by about once per day for at least two days, or about 2 to 7 days, or about 2 days.

In some embodiments, the alpha thymosin is administered for about 1 to 8 weeks, about 1 to 6 weeks, about 1 to 5 weeks, about 1 to 4 weeks, about 2 to 4 weeks, or about 1 -2 weeks. In some embodiments, the thymosin is administered for 1 week, 2 weeks, 3 weeks, 4 weeks, 5, weeks, 6 weeks, 7 week, 8 weeks or more. In some embodiments, the thymosin is administered for about 1 month, 2 months, 3 months or 4 months or more. In some embodiments, the alpha thymosin peptide is administered for about 1 to 4 months, 1 to 3 months, 1 to 2 months, or about one month.

In some embodiments, the alpha thymosin peptide is administered about 1 to 8 times per day for about 1 to 8 weeks. In some embodiments, the alpha thymosin peptide is administered about 1 to 7 times per day, 1 to 6 times per day, 1 to 5 times per day, 1 to 4 times per day, 1 to 3 times per day, 1 to 2 times per day, or about 1 times per day for about 1 to 7 weeks, 1 to 6 weeks, 1 to 5 weeks, 1 to 4 weeks, 1 to 3 weeks, 1 to 2 weeks, or about 1 weeks. In some embodiments, the alpha thymosin peptide is administered about 1 to 8 times per day, 1 to 7 times per day, 1 to 6 times per day, 1 to 5 times per day, 1 to 4 times per day, 1 to 3 times per day, 1 to 2 times per day, or about 1 times per day for about 1 to 30 days, 1 to 25 days, 1 to 20 days, 1 to 15 days, 1 to 7 days or 1 to 5 days. In some embodiments, the alpha thymosin peptide is administered for 1 to 4 times per day for 1 to 30 days. In some embodiments, the alpha thymosin peptide is administered for about 1-2 times per day for 1 to 15 days or 1 to 7 days or 1 to 5 days. In some embodiments, the alpha thymosin peptide is administered about 1 to 2 times per day for 5 days followed by once per day for 2 days. In some embodiments, the alpha thymosin peptide is administered about twice daily for 5 days followed by once per day for 2 days. In some embodiments, the alpha thymosin is administered about four times per day for 5 days or 7 days.

In some embodiments, the regimen employs a dosage of alpha thymosin peptide that is at least 0.2 mg, 0.5 mg, 0.8 mg, 1.6 mg, 3.2 mg, or 6.4 mg, with 1, 2, 3, 4, 5, 6, 7 or 8 or more. In some embodiments, 3 doses or less can be administered. In some embodiments more dosages may be administered, such as 5, 6, 7, 8, 9 or 10, 11, 12, 13, 14, 15, 20, 25, 30, 35, 40, 45, 50 or more. In some embodiments, the dose of thymosin is a relatively low dose of at least 0.2 mg, 0.4 mg, 0.5 mg, 0.8 mg, or 1.6 mg,. The alpha thymosin administrations may be spaced apart by about 1, 2, 3, 4, 5, 6, 7, 8, 9, 10, 12, 16, 20 or 24 hours or about 1, 2, 3, 4, 5, 6, 7, 8, 9 or 10 days, and may be given weekly in some embodiments, as is described in greater detail herein. In some embodiments, the thymosin peptide (e.g., TA1) is administered at a dose within the range of about 0.5 mg to 3 mg. In some embodiments, the thymosin peptide (e.g., TA1) is administered at a dose within the range of about 1 mg to 2 mg.

In some embodiments the thymosin peptide is administered at a dosage of about 0.5 mg, about 0.8 mg, about 1.6 mg, about 3 mg, about 3.2 mg, about 5 mg, or about 6.4 mg or more of thymosin peptide and optionally in combination with one or more treatment schedules described in this paragraph. In some embodiments, the alpha thymosin peptide is administered 1, 2, 3, 4 or more times per day for 1, 2, 3, 4, 5, 6, 7, 8, 9, or 10, 11, 12, 13, 14, 15, 16, 17, 18 ,19, 20, 25 or 30 days or more. In some embodiments, the alpha thymosin peptide is administered for 1, 2, 3, 4, 5, 6, 7 or 8 weeks or more. In some embodiments, the thymosin is administered for 1 or 2 months or more. In some embodiments, the thymosin peptide is administered 1, 2, 3, 4 or more times per day for 2, 3, 4, 5, 6, 7 or 8 days. In some embodiments, the thymosin peptide is administered 1, 2, 3, 4 or more times per day for 4, 5, 6 or 7 days. In some embodiments, the thymosin peptide is administered 1, 2, 3, 4 or more times per day for 5, 6, or 7 days. In some embodiments, the thymosin peptide is administered 1, 2, 3, 4 or more times per day for 1, 2, 3, 4, 5, 6, 7, 8, 9, or 10 days or more, followed by 1, 2, 3, 4 or more times per day for 1, 2, 3, or 4 days or more. In some embodiments, the thymosin peptide is administered 1, 2, 3, 4 or more times per day for 2, 3, 4, 5, 6, 7, or 8 days, followed by 1, 2, 3, 4 or more times per day for 1, 2, 3, or 4 days. In some embodiments, the thymosin peptide is administered 1, 2, 3, 4 or more times per day for 4, 5, 6 or 7 days, followed by 1, 2, 3, 4 or more times per day for 1, 2, 3, or 4 days. In some embodiments, the thymosin peptide is administered 1, 2 or 3 times per day for 4, 5, 6 or 7 days, followed by 1, 2 or 3 times per day for 1, 2, 3, or 4 days. In some embodiments, the thymosin peptide is administered 2 times per day for 7 days. In some embodiments, the thymosin peptide is administered 2 times per day for 5 days. In some embodiments, the thymosin peptide is administered 1 time per day for 5 days. In some embodiments, the thymosin peptide is administered 2 times per day for 5 days, followed by once per day for 2 days. In some embodiments, about 1.6 mg of the thymosin peptide is administered 2 times per day for 5 days, followed by once per day for 2 days.

The timing of thymosin administration may be selected to enhance the immune response including antibody titers (e.g., the development or level of antibody titers) to cover a period of increased risk of sepsis. For example, in certain embodiments, the thymosin peptide administrations are given about 5 days to about 9 days apart, and in various embodiments are administered about 1, 2, 3, 4, 5 6, 7, or 8 days apart. The thymosin administrations may be given about 7 days apart (*e.g*., approximately weekly administration). In other embodiments, the thymosin peptide administrations are given 1, 2, 3, or 4 days apart

In other embodiments, the regimen can be initiated at about 1 to 10 days (in some embodiments 5 to 9 days) prior to an event predicted (or with a significant risk) to result in sepsis, in order to provide for treatment/prevention of sepsis. Exemplary events are described herein. In some of these embodiments, the efficient regimen involves from about 1 to 5 administrations of alpha thymosin peptide, such as 3 or less. The alpha thymosin peptide administrations may be spaced apart by about 1, 2, 3, 4, 5, 6, 7, 8, 9 or 10 days, and may be given weekly in some embodiments.

In some embodiments, the alpha thymosin peptide is first administered prior to an event (as described), such as admittance to a healthcare facility, scheduled surgery, or placement of invasive medical device, and again on the day of the event, and optionally after the event. For example, thymosin peptide may be administered from 1 to 10 days prior to the event, such as from about 5 to about 9 days prior to the event, and again on the day of the event. The thymosin peptide may be administered about 7 days prior to the event, and again on the day of the event, and optionally within 2 to 10 days after the event (*e.g*., from 4 to 8 days after the event). For example, patients receiving two doses of TA1 in accordance with certain embodiments of the invention are likely to achieve a faster and/or larger response to sepsis, and which may be protective for at least 21 days, at least 42 days, or longer.

In some embodiments, the alpha thymosin peptide is administered prior to, along with and/or after an event predicted to result in pathogen exposure or introduction of an opportunistic environment, as described herein. For example, the event may be admittance to a hospital or health care facility for a period of time (*e.g*., at least 3 days, at least one week, or at least ten days, or at least one month). In other embodiments, the event is a scheduled surgery or invasive medical procedure, as described. In other embodiments, the event is the placement of an invasive medical device as described. In still other embodiments, the event is kidney dialysis or initiation of chemotherapy or radiation therapy for cancer treatment (as described).

In some embodiments, the thymosin is administered within the first about 1 hour, 2 hours, 4, hours, 6 hours, 8 hours, 10 hours, 12 hours, 24 hours, 72 hours, 96 hours, 120 hours, 144 hours, or 168 hours, of a determination of sepsis. In some embodiments, the alpha thymosin peptide is administered within the first about 10 minutes, 15 minutes, 30 minutes, 45 minutes or 60 minutes of a determination of sepsis.

In still other embodiments, the regimen involves from 1 to 4 administrations of alpha thymosin peptide, such as 3 or less, and the regimen is timed to begin prior to an event anticipated to lead to sepsis. For example, the regimen may be initiated from 2 to 10 days prior to the event, such as from 5 to 10 days prior, and a second dose may be administered on the day of the event. The alpha thymosin peptide administrations may be spaced apart by about 1, 2, 3, 4, 5, 6, 7, 8, 9 or 10 days, and may be given weekly in some embodiments. In still other embodiments, the regimen involves a dose of alpha thymosin peptide, provided approximately weekly (*e.g*., every 5 to 9 days), for 2, 3, 4 or more weeks.

In still other embodiments, the patient receives 2 doses of an alpha thymosin peptide (such as 2 mg to 8 mg per dose), and such doses are spaced by about 1, 2, 3, 4, 5, 6, 7, 8, 9, 10, 12, 16, 20 or 24 hours or about 1, 2, 3, 4, 5, 6, 7, 8, 9 or 10 days, or approximately weekly. This regimen may be repeated approximately monthly, or every other month, and may be particularly beneficial for protecting chronically ill and immunodeficient patients from sepsis. In some embodiments, the thymosin peptide (*e.g*., TA1) is administered at a dose within the range of about 0.5 mg to 3 mg. In some embodiments, the thymosin peptide (*e.g*., TA1) is administered at a dose within the range of about 1 mg to 2mg.

In certain aspects of the invention, the alpha thymosin peptide regimen is part of an institutional program to reduce the rate or incidence of sepsis, e.g., hospital-acquired sepsis.

In some embodiments, the regimen of alpha thymosin peptide involves administering the agent to the subject at a dose sufficient to enhance antibody titers, and/or sufficient to speed the development of antibody titers, to pathogen exposure. In some other embodiments, the regimen of alpha thymosin peptide involves a regimen provides serum level of alpha thymosin at about 0.01 to 10.0 ng/ml, 0.1 to 1.0 ng/ml, or 0.05 to 5 ng/ml during treatment. In some embodiments, the peak plasma levels of alpha thymosin peptide is at least about 10ng/ml, 20 ng/ml, 30 ng/ml, 40 ng/ml, 50 ng/ml, 60 ng/ml, 70 ng/ml, 80 ng/ml, 90 ng/ml, or 100 ng/ml. In some other embodiments, the regimen of alpha thymosin peptide involves administering the agent to the subject in a regimen so that the subject's pharmacokinetic (pK) profile is substantially the same, e.g., within at least 60%, 70%, 80%, 90% of the pK profile of a subject treated with 1.6 mg of alpha thymosin twice a day for 5 days and then once a day for 2 days. In another embodiment, the pK profile is increased to greater than 100% of the pK profile of a subject treated with 1.6 mg of alpha thymosin twice a day for 5 days and then once a day for 2 days.

The thymosin peptide may be provided in lyophilized form, and reconstituted with sterile (*e.g*., aqueous) diluent prior to administration. The thymosin peptide (e.g., TA1) may be administered by any effective route, including by subcutaneous injection, intramuscular injection, intravenous injection or infusion, and orally. In certain embodiments, the thymosin peptide is administered by subcutaneous injection or by intravenous infusion. Generally, the scheduled dose of thymosin may be administered as a single dose (*e.g*., injection), or may be spaced out over the course of 24 hours or less, for example, by continuous infusion or repeated injection of subdose, or the like or as described extensively herein. In one embodiment, the scheduled dose of thymosin peptide may be administered as a single injection or as multiple injections.

In some embodiments, the alpha thymosin peptide is administered at a dose twice a day for a period of time and then administered at the same dose once a day for a period of time. For example, according to the present invention, alpha thymosin peptide is administered at about 1.6 mg twice per day for 5 days and then 1.6 mg once a day for two days. In some embodiments, the alpha thymosin peptide is administered at about 1.6 mg three or four times per day for 5 to 7 days and then once or twice a day for 2 or 4 days.

In certain embodiments, the patient receives TA1 at a dose of from 2 to 8 mg (*e.g*., at 0.8, 1.6, 3.2 or 6.4 mg per dose) either once or two times daily, or every other day, for from 3 to 14 days (*e.g*., 3, 5, 7, 10, or 14 days). Such regimen may be timed with respect to an event that places the patient at further risk for exacerbation of the infection or complicating illness, such as those events described herein (*e.g*., surgery, hemodialysis, initiation of cancer treatment, placement of medical device). For example, the event may be scheduled at a time between day 2 and day 10 of the regimen, including day 3, day 5, day 7, or day 10. The regimen may be concurrent with antibacterial, antiviral, or antifungal therapy, including with active agents described herein. In some embodiments, the thymosin is administered within the first 24 hours, 48 hours, 72 hours, 96 hours, 120 hours or 144 hours.

In one embodiment, the patient receives approximately weekly administration of TA1, at a dose between 0.5 and 8 mg (*e.g*., about 0.8, 1.6, 3.2 or 6.4 mg), to protect or reduce the severity of sepsis. The regimen may continue in some embodiments for two to four weeks. Where the patient is part of a healthcare facility's TA1 program, the invention results in a reduced incidence of sepsis, reduced number of days in ICU, and/or reduced antimicrobial therapy.

In accordance with the invention, the alpha thymosin peptide of the present invention is administered to a subject with a regimen sufficient to treat sepsis. The alpha thymosin peptide regimen in some embodiments is an "efficient" regimen. That is, the regimen achieves its goal with relatively few administrations of alpha thymosin peptide and/or by timing the administration of alpha thymosin peptide with events anticipated to result in sepsis. The "event" is not a vaccination, but an exposure or increased susceptibility to the potential infectious agent that has the potential to lead or does lead to sepsis, severe sepsis or septic shock. The efficient regimen of alpha thymosin peptide is relatively convenient and comfortable for the patient, as well as more affordable and effective.

According to the present invention, the alpha thymosin peptide used in methods of the present invention can be administered either alone or in combination with a standard of care for sepsis, or as part of treatment regimen involving the standard of care for sepsis. In some embodiments, the standard of care is activated protein C, corticosteroids, intensive insulin therapy synthetic fluid replacement substance (pentastarch), drotrecognin alfa (activated; DrotAA), volume resuscitation, hydrocortisone and fludrocortisone. (See, *e.g*., Hotchkiss, R. S. and Karl, I. E., The Pathophysiology and treatment of Sepsis, NEJM, 348:2 (2008)).

The terms "subject" and "patient" and derivatives thereof can be used interchangeably for the methods of the present invention.

In certain embodiments, the subject is immunodeficient. An immunodeficient subject (*e.g*., a human subject) exhibits a reduced capacity to fight infectious disease and/or a reduced capacity to respond to pathogen exposure. Examples of such immunodeficient subjects include an elderly patient, newborn, leukemic or neutropenic patient, a patient on hemodialysis (*e.g*., for treatment of chronic renal disease), patient receiving immunosuppressant therapy, AIDS patient, diabetic patient, patient receiving chemotherapy or radiation therapy for cancer, immunodeficiency caused by a genetic defect, malnutrition, drug abuse, alcoholism, or other immunocompromising illness or condition.

In certain embodiments, the immunocompromised subject is elderly. As humans and animals age, their immune response is reduced, and the robustness of the immune response is diminished due to the prevalence of low affinity antibody response. Accordingly, the subject in these embodiments may be a human patient over the age of 45, or over the age of 50. In some embodiments, the subject is a human patient 60 years of age or older, 65 years of age or older, or 70 years of age or older.

In some embodiments, the subject is at risk of hospital-acquired sepsis, severe sepsis or septic shock. Hospital-acquired sepsis, severe sepsis or septic shock is sepsis, severe sepsis or septic shock that develops while hospitalized. Since antibiotics are frequently used within hospitals, the microbes associated with sepsis, severe sepsis or septic shock, and their resistance to antibiotics, can differ from isolates outside of the hospital.

In one aspect of the invention, the regimen of thymosin peptide is administered to treat/prevent sepsis, in a patient at risk for sepsis. According to this aspect, the alpha thymosin peptide regimen is used to prime the patient's immune system to provide a more rapid response to a pathogen exposure, which in some embodiments may be anticipated for the patient based upon a scheduled event, and can prevent sepsis.

For example, the subject may be scheduled for an invasive surgical procedure, and in these embodiments, the alpha thymosin peptide regimen reduces the risk and/or severity of post-surgical sepsis. Generally, invasive medical procedures carry a risk of infection, and exemplary procedures include joint replacement, organ or tissue transplantation or graft, introduction of a prosthesis, tissue removal including a tumor or cancerous tissue, tonsillectomy, appendectomy, splenectomy, thymectomy, kidney removal, amputation, removal of bone marrow, or other invasive medical procedure. In such embodiments, the TA1 regimen may reduce the risk of sepsis.

In certain embodiments, the patient may require assistance from an invasive medical device, which causes exposure of the body to microbes, and introduces an opportunistic environment for sepsis to occur. Thus, the device may lead to increased exposure to potential opportunists and pathogens. Such devices include without limitation, a ventilator, a urinary catheter, an arterial catheter, a feeding tube, i.v., stent, kidney dialysis, or artificial organ. In these embodiments, the alpha thymosin peptide regimen helps to prime the patient's immune system to prevent or reduce the severity of any resulting sepsis, severe sepsis or septic shock.

In certain embodiments, the patient is in need, or is under assistance of a pulmonary ventilator, and the TA1 regimen helps to prime the patient's immune system, and retain the immune system in a primed state, so as to reduce the risk or severity of ventilator-associated pneumonia. Ventilator-associated pneumonia (VAP) occurs in patients on mechanical ventilation through an endotracheal or tracheostomy tube, and results from infection in the alveoli. *Pseudomonas aeruginosa* is the most common gram-negative bacterium causing VAP, and *Pseudomonas* has natural resistance to many antibiotics. Other causative species for VAP include *Klebsiella pneumoniae*, which has natural resistance to some beta-lactam antibiotics such as ampicillin and/or carbapenum, as well as cephalosporins and aztreonam. *Serratia marcescens, Enterobactersp.,* and *Acinetobactersp.* may also be associated with VAP, and can also be resistant to antibiotics. In addition, there is an increasing association between *Staphylococcus aureus* (including MRSA) with VAP.

In some embodiments, the patient is on hemodialysis (*e.g*., due to chronic renal disease), or is scheduled to undergo hemodialysis. Since hemodialysis requires access to the circulatory system, patients undergoing hemodialysis may expose their circulatory system to microbes, which can lead to sepsis. Thus, in certain embodiments the TA1 regimen as described herein is initiated to prepare a patient for hemodialysis.

In some embodiments, the patient is a cancer patient, and is undergoing or scheduled to initiate chemotherapy and/or radiation therapy, which often negatively affects the patient's immune system. Where the patient is undergoing or scheduled to initiate chemotherapy, the chemotherapy is generally one that has deleterious effects on the immune cells, and may include one or more alkylating agents (*e.g*., cisplatin, carboplatin, and ifosfamide), antimetabolite (5-fluorouracil or antifolate), topoisomerase inhibitor (*e.g*., camptothecin, etoposide), or taxane (*e.g*., paclitaxel), among others. In some embodiments, the alpha thymosin peptide regimen is administered to prime the patient's immune system prior to cancer therapy in order to prevent or reduce sepsis.

In one exemplary embodiment, a regimen of alpha thymosin peptide as described herein is provided to leukemic and/or neutropenic patients, thereby preventing or reducing the severity of catheter-related sepsis, severe sepsis or septic shock that can be caused by drug resistant *Streptococcus aureus* (*e.g*., MRSA and VRSA). In another exemplary embodiment, a regimen of alpha thymosin peptide as described herein is provided to bone marrow transplant patients, thereby preventing or reducing the severity of sepsis, such as those commonly caused by aspergillus, candida, or CMV. In still another embodiment, a regimen of alpha thymosin peptide as described herein is provided to organ (*e.g*., kidney) transplant recipients, to thereby prevent organ rejection, which is sometimes a result of CMV based sepsis.

In certain embodiments, the symptoms of sepsis are not present or are minor at the time of initiating the TA1 regimen, but the presence of the microorganism or illness is determined by culture, ELISA, or other diagnostic test. In such embodiments, the regimen of alpha thymosin peptide helps to prime the immune system to more rapidly develop an antibody response capable of resolving the infection. In some embodiments, the alpha thymosin peptide regimen is an efficient regimen that is provided concurrently with the standard antibacterial, antiviral, or antifungal therapy.

A variety of diagnostic tests for sepsis or its related conditions are known in the art and can be employed with the methods of the present invention as such tests would be well known to those of skill in the art. Such tests can include but are not limited to blood tests, other laboratory tests, and imaging scans. Blood tests can include but are not limited to tests for evidence of infection (i.e., presence of bacteria, fungi or viruses), clotting problems, abnormal liver or kidney function, impaired oxygen availability, electrolyte imbalances, depressed immune function (such as decreased levels of monocyte HLA-DR), other laboratory tests. Other laboratory tests can include but are not limited to urine tests (e.g., testing urine for infectious agents), wound secretion tests (e.g., testing wound secretions for infectious agents) and respiratory secretion tests (e.g., testing respiratory secretions such as sputum mucus for infectious agents). Imaging tests can include but are not limited to X-ray (e.g., for visualizing infections in the lungs), computerized tomography (CT; e.g., for visualizing infections in the appendix, pancreas or bowels), ultrasound (e.g., for visualizing infections in the gallbladder or ovaries) and magnetic resonance imaging (MRI; e.g., for identifying soft tissue infections, including abscesses within the spine).

In certain embodiments, the patient (or a patient sample, susceptible site for sepsis, or immediate surrounding environment) has tested positive for the presence of a gram positive or gram negative bacteria, including one or more infectious organisms, including, but not limited to: *Lysteria monocytogenes*, *Pseudomonas sp. (e.g., P. aeruginosa), Serratia marcescens, Clostridium difficile, Staphylococcus aureus, Staphylococcus sp., Acinetobacter spp., Enterococcus sp., Enterobacteria sp., E. coli, Klebsiella sp., Streptococcus (e.g., S. pneumoniae), Haemophilus influenzae*, and *Neisseria meningitidis.* In some embodiments, the infection involves, or an isolate is identified, as a drug resistant or multi-drug resistant microorganism, such as *Staphylococcus aureus, Enterococcus sp., Pseudomonas sp., Klebsiella sp., E. coli,* and/or *Clostridium Difficile.* In certain embodiments, the infectious agent is a drug-resistant *S*. *pneumoniae,* including penicillin-resistant, methicillin-resistant, and/or quinolone-resistant (*e.g*., fluoroquinilone). In certain embodiments, the drug-resistant microorganism is methicillin-resistant or vancomycin-resistant *Staphylococcus aureus* (MRSA or VRSA), including intermediate resistant isolates, or is carbapenum-resistant *E. coli, Klebsiella,* or *Pseudomonas* including intermediate resistant isolates. The presence of such organisms may be determined or confirmed using diagnostics tests known in the art, or determined by a spike in the incidence of such infection at the healthcare facility.

In particular exemplary embodiments, the patient is a neutropenic patient inflicted with a *Pseudomonas*, *Acinetobacter*, or *E. coli* infection, and the resulting sepsis, severe sepsis or septic shock may be due drug resistant microorganisms, or the patient is inflicted with ventilator-associated pneumonia, which may involve infection with *Pseudomonas* or *Serratia,* which may also lead to drug resistant sepsis, severe sepsis or septic shock.

The regimen of alpha thymosin peptide may be administered concurrently with antibiotic therapy, including with beta-lactam antibiotic (*e.g*., methicillin, ampicillin, carbapenem, piperacillin); cephalosporin; fluoroquinolone (*e.g*., ciprofloxacin, levofloxacin, moxifloxacin), and/or macrolide (*e.g*., azithromycin, clarithromycin, dirithromycin, and erythromycin). The antibiotic therapy may be administered with additional therapeutics, such as a beta-lactamase inhibitor (tazobactam). In certain embodiments, alpha thymosin peptide reduces the duration of sepsis, and reduces the duration of required antibiotic treatment. In certain embodiments, the infection is determined to be resistant to such agent, prior to initiating alpha thymosin peptide treatment. In certain embodiments, the alpha thymosin peptide regimen is initiated, or continued, or repeated, after apparent resolution of sepsis, to help prevent recurrence after antibiotic therapy is complete. An efficient regimen of alpha thymosin peptide (*e.g*., 1, 2, 3, 4, 5, or 6 doses) may span the full course of antibacterial therapy, and provide a boost in immune response for the entire period.

In certain embodiments, the patient has sepsis resulting from a viral infection selected from cytomegalovirus (CMV), RSV, influenza virus, herpes simplex virus type 1, and parainfluenza virus. The alpha thymosin peptide regimen described herein may reduce the severity and/or duration of the viral based sepsis, severe sepsis or septic shock, and may be provided alongside the appropriate antiviral therapy, which may be a virus-neutralizing antibody or a small molecule inhibitor, such as Tamiflu. In certain embodiments, the alpha thymosin peptide regimen is initiated, or continued, or repeated, after apparent resolution of the viral based sepsis, severe sepsis or septic shock, to help prevent recurrence after other therapy is complete.

In still other embodiments, the patient has a sepsis resulting from a fungal infection of *Aspergillus* (*e.g., A. fumigatus)* or *Candida* (*e.g.*, *Candida albicans*), and these may also show resistance to antibiotic treatments. In certain embodiments, the thymosin peptide regimen is administered with antifungal treatment. Antifungal therapies include azole drug such as an imidazole (*e.g*., ketoconazole) or a triazole (*e.g*. fluconazole). In certain embodiments, the alpha thymosin peptide regimen is initiated, or continued, or repeated, after apparent resolution of the infection, to help prevent recurrence after antifunga

According to some embodiments of the present invention, administering of alpha thymosin peptide according to the methods of the present invention provides statistically significant therapeutic effect. In one embodiment, the statistically significant therapeutic effect is determined based on one or more standards or criteria provided by one or more regulatory agencies in the United States, e.g., FDA or other countries. In another embodiments, the statistically significant therapeutic effect is determined based on results obtained from regulatory agency approved clinical trial set up and/or procedure.

In some embodiments, the statistically significant therapeutic effect is determined based on a patient population of at least 300, 400, 500, 600, 700, 800, 900, 1000 or 2000. In some embodiments, the statistically significant therapeutic effect is determined based on data obtained from randomized and double blinded clinical trial set up. In some embodiments, the statistically significant therapeutic effect is determined based on data with a p value of less than or equal to about 0.05, 0.04, 0.03, 0.02 or 0.01. In some embodiments, the statistically significant therapeutic effect is determined based on data with a confidence interval greater than or equal to 95%, 96%, 97%, 98% or 99%. In some embodiments, the statistically significant therapeutic effect is determined on approval of Phase III clinical trial of the methods provided by the present invention, e.g., by FDA in the US.

In some embodiment, the statistically significant therapeutic effect is determined by a randomized double blind clinical trial of a patient population of at least 300 or 350; treated with alpha thymosin peptides in combination with standard care, but not in combination with any protease inhibitor. In some embodiment, the statistically significant therapeutic effect is determined by a randomized clinical trial of a patient population of at least 300 or 350 and using 28 day mortality rate, in-hospital mortality rate, ICU mortality rate, ICU duration, ICU free days, sequential organ failure assessment score (SOFA), relative risk of death, ICU frequency, duration of ventilation, frequency of ventilation, ventilation free days, HLA-DR expression or any combination thereof or any other commonly accepted criteria for sepsis assessment.

In general, statistical analysis can include any suitable method permitted by a regulatory agency, e.g., FDA in the US or China or any other country. In some embodiments, statistical analysis includes non-stratified analysis, log-rank analysis, e.g., from Kaplan-Meier, Jacobson-Truax, Gulliken-Lord-Novick, Edwards-Nunnally, Hageman-Arrindel and Hierarchical Linear Modeling (HLM) and Cox regression analysis.

In some embodiments, sepsis, severe sepsis or septic shock biomarkers can be used for predicting treatment response and/or determining treatment efficacy. In some embodiments, mHLA-DR can be measured and improvement in mHLA-DR levels employed as an indicator of positive treatment response. In some embodiments, decreased or reduced levels of mHLA-DR biomarker (including protein expression levels, mRNA transcript levels, reduced number of mHLA-DR positive monocytes), which later increase upon administration of an alpha thymosin peptide is predictive of treatment response. In some embodiments, this information can be employed in determining a treatment regimen (as described herein) for the treatment of sepsis, severe sepsis or septic shock using alpha thymosin peptides according to the present invention. As such, the present invention provides methods for determining a treatment regimen which include detecting an increase or decrease in the level of a sepsis, severe sepsis or septic shock biomarker in a biological sample from a subject treated with an alpha thymosin peptide and determining a treatment regimen of the alpha thymosin peptide based on an increase or decrease in the level of one or more one or more sepsis, severe sepsis or septic shock biomarkers in a biological sample. In some embodiments, the sepsis, severe sepsis or septic shock biomarker is mHLA-DR. In some embodiments, a decreased or reduced level of mHLA-DR is indicative of treatment response and/or treatment efficacy of treatment with alpha thymosin for sepsis, severe sepsis or septic shock. In some embodiments, a decreased or reduced level of mHLA-DR which is enhanced or increased in response to alpha thymosin peptide treatment is indicative of treatment response and/or treatment efficacy of treatment with alpha thymosin peptide for sepsis, severe sepsis or septic shock. In some embodiments, recovery of mHLA-DR levels to a predetermined standard level is indicative of better treatment prognosis (e.g., better survival rate) in treatment with an alpha thymosin peptide. In some embodiments, larger increases in mHLA-DR levels is indicative of better treatment prognosis in treatment with an alpha thymosin peptide.

As used herein, the phrase "determining the treatment efficacy" and variants thereof can include any methods for determining that a treatment is providing a benefit to a subject. The term "treatment efficacy" and variants thereof are generally indicated by alleviation of one or more signs or symptoms associated with the disease and can be readily determined by one skilled in the art as the alleviation of one or more signs or symptoms of the indication or disease being treated. "Treatment efficacy" may also refer to the prevention or amelioration of signs and symptoms of toxicities typically associated with standard treatments of a disease, *i.e.* chemotherapy or radiation therapy for the treatment of cancer. Such methods are indication and disease specific and can include any methods well known in the art for determining that a treatment is providing a beneficial effect to a patient. For example, evidence of prevention, treatment or reduction of sepsis, severe sepsis or septic shock, as well as determining treatment response can include decreased incidence of sepsis, severe sepsis or septic shock as well as reduced in-hospital mortality, ICU mortality, increased ICU-free days, reduced number of days in ICU, reduced ICU duration, reduced ICU frequency, beneficial or improved sequential organ failure assessment score (SOFA), reduced duration of ventilation, reduced frequency of ventilation. Treatment efficacy can include but is not limited to remission of the sepsis, severe sepsis or septic shock, including for example, a decrease or reduction in the underlying infection causing the sepsis, severe sepsis or septic shock. Further, treatment efficacy can also include general improvements in the overall health of the subject, such as but not limited to enhancement of patient life quality, increase in predicted subject survival rate, decrease in depression or decrease in rate of recurrence of the indication (increase in remission time). (See, *e.g*., Physicians' Desk Reference (2010) and Dellinger R.P., et al., Surviving Sepsis Campaign: international guidelines for management of severe sepsis and septic shock: 2008; Intensive Care Med. 34(4):783-785, (2008)).

Predetermined standard levels of sepsis, severe sepsis or septic shock biomarkers can be defined using a variety of methods known to those of skill in the art. Generally, standard levels for a biomarker are determined by determining the level of a biomarker in a sufficiently large number of samples obtained from normal, healthy control subjects (*e.g*., subjects not exhibiting sepsis, severe sepsis or septic shock). Further, standard level information can be obtained from publically available databases, as well as other sources. (See, *e.g*., Bunk, D.M., "Reference Materials and Reference Measurement Procedures: An Overview from a National Metrology Institute," Clin. Biochem. Rev., 28(4):131-137 (2007); Suraj Peri1, et al., "Development of Human Protein Reference Database as an Initial Platform for Approaching Systems Biology in Humans" Genome Res. 13: 2363-2371 (2003); Remington: The Science and Practice of Pharmacy, Twenty First Edition (2005).) In some embodiments, an increase or decrease of the level of one or more sepsis, severe sepsis or septic shock markers in a sample obtained from a subject treated with an alpha thymosin peptide is determined by comparing the level of one or more sepsis, severe sepsis or septic shock biomarkers to a predetermined standard level.

Information regarding the increase or decrease in the level of one or more sepsis, severe sepsis or septic shock biomarkers can be used to determine the treatment efficacy of treatment with an alpha thymosin peptide, as well as to tailor treatment regimens for treatment with an alpha thymosin peptide. In some embodiments the treatment efficacy can be used to determine whether to continue treatment with an alpha thymosin peptide. In other embodiments the treatment efficacy can be used to determine whether to discontinue treatment with an alpha thymosin peptide. In other embodiments the treatment efficacy can be used to determine whether to modify treatment with an alpha thymosin peptide. In other embodiments the treatment efficacy can be used to determine whether to increase or decrease the dosage of alpha thymosin peptide that is administered. In other embodiments the treatment efficacy can be used to determine whether to change the dosing frequency. In further embodiments, the treatment efficacy can be used to determine whether to change the number of dosage per day, per week, times per day. In yet further embodiments the treatment efficacy can be used to determine whether to change the dosage amount.

Methods for obtaining biological samples are well known in the art and any standard methods for obtaining biological samples can be employed. Biological samples that find use with the methods of the present invention include but are not limited to serum, blood, plasma, whole blood and derivatives thereof, skin, hair, hair follicles, saliva, oral mucous, vaginal mucous, sweat, tears, epithelial tissues, urine, semen, seminal fluid, seminal plasma, prostatic fluid, pre-ejaculatory fluid (Cowper's fluid), excreta, biopsy, ascites, cerebrospinal fluid, lymph, and tissue extract sample or biopsy. (See, e.g., Clinical Proteomics: Methods and Protocols, Vol. 428 in Methods in Molecular Biology, Ed. Antonia Vlahou (2008).)

### EXAMPLES

### EXAMPLE 1:

### THE EFFICACY OF THYMOSIN ALPHA 1 FOR SEVERE SEPSIS (ETASS): A MULTIICENTER, SINGLE-BLIND. RANDOMIZED AND CONTROLLED TRIAL

### ABSTRACT

INTRODUCTION: Severe sepsis is associated with a high mortality rate despite implementation of guideline recommendations. Adjunctive treatment may be efficient and require further investigation. In light of the crucial role of immunologic derangement in severe sepsis, thymosin alpha 1 (Talpha1) is considered as a promising beneficial immunomodulatory drug. The trial is to evaluate whether Talpha1 improves 28-day all-cause mortality rates and immunofunction in patients with severe sepsis. Methods We performed a multicenter randomized controlled trial in 6 tertiary, teaching hospitals in China between May 12, 2008 and Dec 22, 2010. Eligible patients admitted in ICU with severe sepsis were randomly allocated by a central randomization center to control group or Talpha1 group (1:1 ratio). The primary outcome was death from any cause and was assessed 28 days after enrollment. Secondary outcomes included dynamic changes of Sequential Organ Failure Assessment (SOFA) and mHLA-DR (monocyte human leukocyte antigen-DR) on day 0, 3, 7 in both groups. All analyses were done on an intention-to-treat basis. RESULTS: 361 patients were allocated to either the control group (n=180) or Talpha1 (n=181) group. The mortalities from any cause within 28 days in Talpha1 group and control group were 26.0% and 35.0% respectively with a marginal p-value (nonstratified analysis, p=0.062; log-rank, p=0.049); the relative risk of death in the Talpha1 group as compared to the control group was 0.74 (95% CI 0.54-1.02). Greater improvement of mHLA-DR was observed in Talpha1 group on day 3 (mean difference in mHLA-DR changes between the two groups was 3.9%, 95% CI 0.2-7.6%, p=0.037) and day 7 (mean difference in mHLA-DR changes between two groups was 5.8%, 95% CI 1.0∼10.5%, p=0.017) than in control group. No serious drug-related adverse event was recorded.

CONCLUSIONS: The use of Talpha1 therapy in combination with conventional medical therapies may be effective in improving clinical outcomes in a targeted population of severe sepsis.

Trial registration: ClinicalTrials.gov NCT00711620.

### INTRODUCTION

Severe sepsis is an important cause of admission to intensive care units (ICUs) throughout the world and is characterized by high mortality in adults [1-3]. Severe sepsis is diagnosed in more than 750,000 people annually in the United States, of whom 215,000 will die [3]. Reported mortality rates of severe sepsis ranged from 28% to 35.5% [3-7]. In spite of the adoption of therapeutic bundles based on Surviving Sepsis Campaign (SSC) guidelines, mortality is reported to be about 30% [4]. The key role of immunologic derangement in the course and the poor outcome has led to an increased interest in immunotherapy [8,9]. Thymosin alpha 1 (Tα1) is a naturally occurring thymic peptide first described and characterized by Goldstein et al. [10]. It acts as an endogenous regulator of both the innate and adaptive immune systems [11]. It is used worldwide for treating diseases associated with immune dysfunction including viral infections such as hepatitis B and C, certain cancers, and for vaccine enhancement [12,13]. Notably, recent development in immunomodulatory research has indicated the beneficial effect of Tα1 treatment in septic patients. However, the results of these studies should be viewed with caution due to their small sample sizes and use of more than one drug as therapeutic intervention [14-16]. This multicenter randomized controlled trial was implemented to determine the efficacy of Tα1 in treating severe sepsis.

### MATERIAL AND METHODS

We did a prospective, controlled, single-blinded, multi-center randomized clinical trial, which was conducted in the ICUs of six tertiary, teaching hospitals. The ethics committee of the First Affiliated Hospital of Sun Yat-sen University approved the protocol (200815). Written informed consents were obtained from the patients or next of kin for patients unable to consent. The trial was registered with ClinicalTrials.gov, number NCT00711620.

### Patients

From May 12, 2008 to Dec 22, 2010 patients diagnosed with severe sepsis admitted to ICUs were enrolled in the trial. The criteria for severe sepsis were a modification of those defined by Bernard et al. (see Additional file 1) [7]. Patients were eligible for study inclusion if they had a known or suspected infection based on clinical data at the time of screening and if they had two or more signs of systemic inflammation and sepsis-induced dysfunction of at least one organ or system.

### Randomization and masking

To reduce the impact on the results from heterogeneity of severe sepsis and inter-hospital variation in patient sources as much as possible, stratification by investigative center in combination with computer-generated block randomization (block size = 8) according to the sequence of recruitment was employed in the enrollment process. The method of randomization and block size were blinded until the data analysis was finished completely. Clinicians who enrolled the subjects were not involved in data collection. Eligible patients were randomly assigned in a 1:1 ratio in each hospital with four in each block assigned to receive the study drug and the other four to the control group after telephone verification through a randomization center. The allocation sequence was concealed from the researchers. To prevent advance knowledge of treatment assignment and subversion of the allocation sequence, trial entry sheet of the case report form (CRF) was filled out and informed consent was obtained before disclosing the unique participant number and the allocated group; the unique number generated could not be changed and deleted afterward. We used normal saline as placebo. Patients were blinded to the treatment assignments. All statistical analysis was done with masking maintained.

### Study drug administration and sepsis management

In the Tα1 group, patients received subcutaneous injections of 1.6 mg Tα1 (ZADAXIN ™, SciClone Pharmaceuticals, Foster City, CA, USA) twice per day for five consecutive days, then once per day for two consecutive days. Prior to administration, the lyophilized powder is to be reconstituted with 1 ml of the provided diluent (sterile water for injection). After reconstitution, the final concentration of Tα1 is 1.6 mg/mL. In the control group, patients received subcutaneous injections of 1 mL normal saline twice per day for five consecutive days, then once per day for two consecutive days. According to trial protocol, therapy had to be started within 4 hrs after enrollment.

The treating physicians dictated patient care to current international guidelines [17], including adequate empiric antibiotic therapy based on current recommendations, ventilation regimen (pressure control mode), blood glucose control, resuscitation and hemodynamic support, organ support, sedation or analgesia as needed and adequate nutrition. Empirical antibiotic therapy was considered adequate when at least one effective drug was included in the empirical antibiotic treatment within the first 24 hrs of the admission to the ICU and the optimal dose and the correct route of administration were in accordance with medical standards and in ICU survivors without microbiologically detected microorganism in bloodstream or focus. When the empirical antibiotic therapy had to be changed after microbiological detection of microorganism, it was considered inadequate, whereas in non-survivors without microbiologically detected microorganism in bloodstream or focus it was considered not evaluable [18-20].

### Outcomes and data collection

The primary efficacy end point was death from any cause and was assessed 28 days after the initiation of treatment assignment. Secondary outcomes included dynamic changes of Sequential Organ Failure Assessment (SOFA), CD 4+/CD8+ and monocyte human leukocyte antigen-DR (mHLA-DR) expression measured on day 0 (the day of enrollment), 3 and 7 in both groups. All mHLA-DR measurements were done in the center laboratory of the First Affiliated Hospital of Sun Yat-sen University. 1 ml unprocessed EDTA whole blood was stored on ice at once after drawing and was transferred to the center laboratory as soon as possible to guarantee measurement within 3 hrs after blood drawing. The method of measuring mHLA-DRwas mentioned in our previous paper [21]. Once patients were enrolled, data including demographic characteristics, microbiological findings (primary infection source and the identified microorganisms) and comorbidities were collected when available. The following clinical parameters were recorded on specific days after enrollment: on day 0, the severity as assessed by the Acute Physiology and Chronic Health Evaluation II (APACHE II); on day 0, 3, 7, SOFA, hematologic and biochemical findings, results of mHLA-DR, CD4+/CD8+ tests. The time of the first organ dysfunction was retrospectively estimated according to objective data such as blood gas analysis when the patient was enrolled.

### Statistical analysis and sample size

Based on a previous study [22], a sample size of 334 patients was required to show a reduction in 28-day mortality rate from 50% to 35% by Tα1 treatment, with a two-sided test (a error = 5%; power = 80%). Considering a possible drop-out rate of 10%, the trial would need to enroll 368 patients in total. Demographic data, outcome data and other laboratory parameters were summarized by frequency for categorical variables and mean ± standard deviation (SD) or median with interquartile range (IQR) for continuous variables. Proportions were compared with chi-square test or Fisher's exact test. Continuous variables were tested by means of t test with normal distribution or Wilcoxon rank-sum test with non-normal distribution. The comparison of primary outcome between two groups was performed by means of Cochran-Mantel-Haenszel test, in which patients were stratified on a number of baseline covariates such as mHLA-DR, scores of APACHE and SOFA, surgical and cancer history, sex and age. The corresponding relative risks (RRs) with 95% confidence intervals (CIs) were computed with logit-adjusted method. Kaplan-Meier estimates without adjustment for baseline covariates were used for survival time analysis, and log-rank tests for comparison. To estimate mean changes from baseline in laboratory parameters, linear mixed models for repeated measures were employed, taking into account the clustering of participating centers and repeated measurements within patients. This model included terms for baseline measurement, treatment group, visit, and treatment × visit interaction. Least-squares means with 95% CIs were reported. We also analyzed the efficacy parameters of the study drug in different prespecified subgroups. The heterogeneity of treatment effects among subgroups was assessed with use of interaction tests. Consistent with the intention-to-treat principle, all analyses were based on all available population, consisting of those with a baseline and at least one post-baseline efficacy measurement, neither making any assumption nor imputing the missing data. All statistical analyses were done with the SAS software (SAS 9.1.3; SAS Institute Inc., Cary, NC, USA). Two-sided P values were reported and a P value less than 0.05 was considered as statistically significant.

### RESULTS

### Study profile

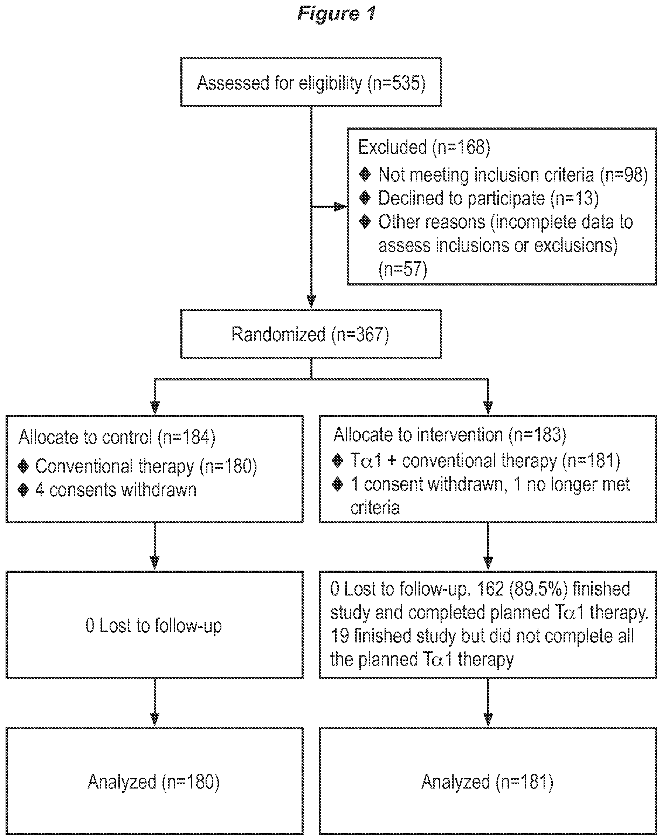

Between May 12, 2008 and Dec 22, 2010, 367 eligible patients were randomized (Figure 1). In the Tα1 group, two patients were excluded: one patient withdrew the consent after being diagnosed with typhus and was transferred to the infectious disease hospital immediately; in the other case, consent was withdrawn before the infusion. In the control group, consents were withdrawn after the enrollment in four cases. A total of 361 randomized patients were followed up for the entire 28-day study period without drop-out. Of 181 patients in Tα1 group, 162 patients completed the trial in adherence with the protocol regarding the use of drugs, while the other 19 patients received at least 1.6 mg Tα1 but their treatments did not fully adhere to the protocol because they were transferred out of ICU.

### Baseline data

Both groups had similar characteristics in most demographic and baseline variables (Table 1), although patients in the Tα1 group had a longer period between the time of first organ dysfunction observed and the time of enrollment (42 hrs vs. 28 hrs, P = 0.003). Nearly 80% of the patients had at least two dysfunctional organs at the time of enrollment. The pulmonary and cardiovascular systems were the most commonly affected organ systems with an incidence of 94.7% and 65.7% respectively. The most common sites of infection were lung and abdomen, with an incidence of 74.5 and 27.4%, with mixed pathogens or gram-negative organisms accounting for the majority of cases. There was no difference in adequate antibiotic treatment (refer to Table 2). Baseline laboratory data were comparable between the two groups and shown in Table 3. Patients in the Tα1 group had a lower level of mHLA-DR (47.1 vs. 58.0% in the control group, P = 0.02), but the distribution of each stratum in the two groups was similar.

**Table 1: Baseline characteristics in both study groups.**

| | **Control group** | **TαI group** | ***P* value** |
|---|---|---|---|
| n | 180 | 181 | |
| Age (yr) | 66.4 ± 12.6 | 64.7 ± 14.5 | 0.46 |

| Age group | | | |
|---|---|---|---|
| < 50 yr | 21 (11.7%) | 24 (13.3%) | |
| 50-60 yr | 39 (21.7%) | 45 (24.9%) | |
| 61-70 yr | 39 (21.7%) | 39 (21.6%) | |
| 71-yr | 81 (45.0%) | 73 (40.3%) | |
| Male | 131 (72.8%) | 141 (77.9%) | 0.26 |
| Female | 49 (27.2%) | 40 (22.1%) | |
| BMI | 22.0 ± 3.0 | 22.2 ± 3.1 | 0.48 |

| **Prior or preexisting conditions** | | | |
|---|---|---|---|
| Congestive heart failure | 8 (4.4%) | 5 (2.8%) | 0.39 |
| Hypertension | 79 (43.9%) | 80 (44.2%) | 0.95 |
| Coronary heart disease | 19 (10.6%) | 22 (12.2%) | 0.63 |
| Liver disease | 10(5.6%) | 9 (5.0%) | 0.80 |
| COPD | 28 (15.6%) | 29 (16.0%) | 0.90 |
| Nervous system diseases | 33 (18.3%) | 32 (17.7%) | 0.87 |
| Diabetes | 34(18.9%) | 40 (22.1%) | 0.45 |
| Recent trauma | 8(4.4%) | 8 (4.4%) | 0.99 |
| Cancer | 55 (30.6%) | 60 (33.2%) | 0.60 |

| **Recent surgical history** | | | 0.47 |
|---|---|---|---|
| No history of surgery | 103 (57.2%) | 92 (50.8%) | |
| Elective surgery | 41 (22.8%) | 46 (25.4%) | |
| Emergency surgery | 36 (20.0%) | 43 (23.8%) | |

| **Other indicators of disease severity** | | | |
|---|---|---|---|
| Mechanical ventilation | 143 (79.4%) | 146 (80.7%) | 0.77 |
| Shock | 74(41.1%) | 64 (35.4%) | 0.26 |
| Use of any vasopressor or dobutamine | 72 (40.0%) | 71 (39.2%) | 0.88 |
| Low-dose corticoid | 18 (10.0%) | 20 (11.1%) | 0.75 |
| Blood transfusion | 54 (30.0%) | 64 (35.4%) | 0.28 |

| **Baseline acute organ dysfunctions** | | | |
|---|---|---|---|
| Pulmonary | 170 (94.4%) | 172 (95.0%) | 0.80 |
| Renal | 48 (26.7%) | 53 (29.3%) | 0.58 |
| Cardiovascular | 113 (62.8%) | 124 (68.5%) | 0.25 |
| Hematologic | 69 (38.3%) | 67 (37.0%) | 0.80 |
| Hepatic | 39(21.7%) | 27(14.9%) | 0.10 |

| **Number of acute organ dysfunction** | | | 0.97 |
|---|---|---|---|
| 1 | 32(17.8%) | 29(16.0%) | |
| 2 | 75 (41.7%) | 77 (42.5%) | |
| 3 | 45 (25.0%) | 48 (26.5%) | |
| 4 | 18 (10.0%) | 19 (10.5%) | |
| 5 | 10 (5.6%) | 8 (4.4%) | |
| **APACHE II score** | 21.6 ± 7.7 | 22.3 ± 6.7 | 0.35 |
| **SOFA score** | 7.7 ± 3.9 | 7.9 ± 3.6 | 0.65 |
| Respiratory system | 2.6 ± 1.0 | 2.7 ± 0.9 | 0.22 |
| Coagulation | 0.8 ± 1.1 | 1.0 ± 1.2 | 0.17 |
| Cardiovascular system | 1.4 ± 1.6 | 1.2 ± 1.5 | 0.40 |
| Liver | 0.6 ± 0.9 | 0.5 ± 0.8 | 0.38 |
| Nervous system | 1.3 ± 1 .4 | 1.4 ± 1.4 | 0.69 |
| Renal system | 1.0 ± 1.3 | 1.0 ± 1.4 | 0.85 |
| Time from first organ dysfunction to enrollment (hr) median(IQR) | 28.0 (15.0-48.0) | 42.0 (24.0-72.0) | 0.003 |

| | | | |
|---|---|---|---|
| Apache II, Acute Physiology and Chronic Health Evaluation II; BMI, body mass index; COPD, chronic obstructive pulmonary disease; IQR, interquartile range; SOFA, Sequential Organ Failure Assessment; TαI, thymosin alpha 1; yr, year. | | | |

**Table 2: Sites, causes of infection and adequate antibiotic treatment in patients with severe sepsis.**

| | **Control group (*n* = 180)** | **TαI group (*n* = 181)** | ***P* value** |
|---|---|---|---|
| **Sites of infection*** | | | |
| Lung | 133 (73.9%) | 136 (75,1%) | 0.79 |
| Abdomen | 48 (26.7%) | 51 (28.2%) | 0.75 |
| Urinary tract | 5 (2.8%) | 2 (1.1%) | 0.28 |
| Positive blood culture | 10 (5.6%) | 11 (6.1%) | 0.83 |
| Other^{†} | 18 (10.0%) | 16 (8.8%) | 0.71 |
| **Results of pathogens** | | | 0.99 |
| Pure gram-negative | 47 (26.1%) | 51 (28.2%) | |
| Pure gram-positive | 15 (8.3%) | 14 (7.7%) | |
| Pure fungus | 22 (12.2%) | 21 (11.6%) | |
| Mixed | 57 (31.7%) | 56 (30.9%) | |
| Culture negative | 39 (21.7%) | 39 (21.6%) | |

| **Types of organisms^{‡}** | | | |
|---|---|---|---|
| **Gram-positive** | | | |
| Staphylococcus aureus | 7 (3.9%) | 9 (5.0%) | 0.62 |
| Other staphylococcus species | 9 (5.0%) | 12 (6.6%) | 0.51 |
| Enterococcus species | 22 (12.2%) | 23 (12.7%) | 0.89 |
| Other gram-positive | 18 (10.0%) | 14(7.7%) | 0.45 |

| **Gram-negative** | | | |
|---|---|---|---|
| Klebsiella species | 18 (10.0%) | 22 (12.2%) | 0.51 |
| Escherichia coli | 25 (13.9%) | 23 (12.7%) | 0.74 |
| Pseudomonas species | 32 (17.8%) | 32 (17.7%) | 0.98 |
| Acinetobacter | 8 (4.4%) | 15 (8.3%) | 0.14 |
| Enterobacter species | 4 (2.2%) | 4 (2.2%) | 1.00 |
| Other gram-negative | 14 (7.8%) | 16 (8.8%) | 0.71 |

| **Fungus** | | | |
|---|---|---|---|
| Candida albicans | 43 (23.9%) | 38 (21.0%) | 0.51 |
| Other candida species | 20 (11.1%) | 15 (8.3%) | 0.36 |
| Mould | 1 (0.6%) | 4 (2.2%) | 0.37 |
| Other fungus | 6 (3.3%) | 6 (3.3%) | 0.99 |

| **Empirical antibiotic therapy** | | | 0.903 |
|---|---|---|---|
| Adequate | 136 (75.6%) | 133 (73.5%) | |
| Inadequate | 34 (18.9%) | 37 (20.4%) | |
| Not evaluable | 10 (5.6%) | 11 (6.1%) | |

| | | | |
|---|---|---|---|
| *Patients may have had more than one site of infection; ^{†}other sites of infection included skin, central nervous system, bones and joints; ^{‡}patients may have had more than one organism cultured. Tα1, thymosin alpha 1. | | | |

**Table 3: Baseline levels of laboratory values.**

| | **Control group** | **TαI group** | ***P* value** |
|---|---|---|---|
| mHLA-DR (%) | | | |
| Median (IQR) | 58.0 (33.9-83.0) | 47.1 (26.4-71.1) | 0.02 |

| mHLA-DR group | | | 0.16 |
|---|---|---|---|
| < 30% (n, %) | 36 (20.3%) | 50 (27.6%) | |
| ≥ 30- < 45% (n, %) | 29 (16.4%) | 32 (17.7%) | |
| ≥ 45- < 85% (n, %) | 70 (39.6%) | 71 (39.2%) | |
| ≥ 85% (n, %) | 42 (23.7%) | 28 (15.5%) | |

| CD⁴⁺/CD⁸⁺ | | | |
|---|---|---|---|
| Median (IQR) | 1.95 (1.18-3.30) | 1.87 (1.16-3.22) | 0.64 |

| WBC (*10⁹) | | | |
|---|---|---|---|
| Median level | 14.3 (10.1-17.9) | 14.4 (9.4-19.3) | 0.78 |

| Neutrophil (%WBC) | | | |
|---|---|---|---|
| Median (IQR) | 85.1 (80.2-90.7) | 86.5 (80.8-91.0) | 0.48 |

| Lymphocyte (%WBC) | | | |
|---|---|---|---|
| Median (IQR) | 9.5 (6.0-15.3) | 8.9 (5.0-14.1) | 0.23 |

| Monocyte (%) | | | |
|---|---|---|---|
| Median (IQR) | 4.80 (3.30-7.30) | 4.95 (2.80-7.30) | 0.66 |

| Lactate (mmol/L) | | | |
|---|---|---|---|
| Median (IQR) | 2.1 (1.4-3.4) | 2.1 (1.3-3.1) | 0.86 |

| | | | |
|---|---|---|---|
| CD, cluster of differentiation; CI, confidence interval; mHLA-DR, monocyte human leukocyte antigen-DR; SOFA, Sequential Organ Failure Assessment; Tα1, thymosin alpha 1. | | | |

### STUDY OUTCOMES

### Primary outcome

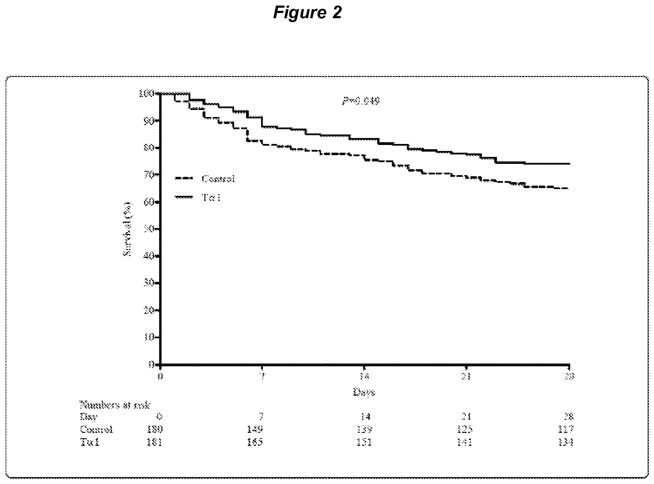

Within 28 days after the enrollment, 47 of 181 patients in the Tα1 group (26.0%) and 63 of 180 patients in the control group (35.0%) expired. The relative risk of death in the Tα1 group as compared to the control group was 0.74 (95% CI 0.54 to 1.02) with a P value of 0.062 in the non-stratified analysis. There was a 9.0% (95% CI -0.5 to 18.5%) absolute reduction in mortality in the Tα1 group. Survival time-to-event curves of the two groups are presented in Figure 2. Patients in the Tα1 group survived longer after enrollment than the control group (log rank, P = 0.049). A total of 52 of 181 patients in the Tα1 group (28.7%) and 71 of 180 patients in the control group (39.4%) died in hospital. The relative risk of death in hospital in the Tα1 group was 0.73 (95% CI 0.54 to 0.98) compared to the control group with a P value of 0.032. There was no significant difference in ICU mortality, ventilation-free days, ICU-free days, the length of ICU stay and duration of mechanical ventilation between the two groups (Table 4).

**Table 4 Primary outcome and prognosis.**

| | **Control group (*n* = 180)** | **TαI group (*n* = 181)** | ***P* value** |
|---|---|---|---|
| 28-day mortality | 63 (35.0%) | 47 (26.0%) | 0.062 |
| In-hospital mortality | 71 (39.4%) | 52 (28.7%) | 0.032 |
| In-ICU mortality | 48 (26.7%) | 35 (19.3%) | 0.098 |

| Duration of ventilation | | | |
|---|---|---|---|
| Median (IQR) | 6.0 (2.0-14.0) | 7.0 (3.0-13.0) | 0.742 |

| ICU stay | | | |
|---|---|---|---|
| Median (IQR) | 10.5 (5.0-20.5) | 11.0 (7.0-20.0) | 0.254 |

| Ventilation-free days* | | | |
|---|---|---|---|
| Median (95% CI) | 13.0 (7.0-18.0) | 18.0 (15.0-21.) | 0.077 |

| ICU-free days* | | | |
|---|---|---|---|
| Median (95% CI) | 5.0 (0.3-10.7) | 10.0 (6.8-15.0) | 0.235 |

| | | | |
|---|---|---|---|
| 'Free days' were calculated as the number of days that the patient was alive and free of given measure (ventilator use and ICU stay) during the 28-day study period. CI, confidence interval; IQR, interquartile range, Tα1, thymosin alpha 1. | | | |

### Secondary outcomes

Dynamic changes in SOFA and laboratory measurements are summarized in Table 5. A sustained increase in mHLA-DR values (% of positive monocytes) was observed in both groups. The mean changes from baseline on day 3 and day 7 were 4.1% and 11.2% in the control group, and 8.0% and 17.0% in the Tα1 group. Patients in the Tα1 group had lower baseline mHLA-DR than those in the control group on day 0. Greater improvements in mHLA-DR were observed in patients in the Tα1 group on day 3 (mean difference in mHLA-DR changes between the two groups was 3.9%, 95% CI 0.2 to 7.6%, P = 0.037) and day 7 (mean difference in mHLA-DR changes between two groups was 5.8%, 95% CI 1.0 to 10.5%, P = 0.017). The average SOFA score changes on day 3 and day 7 were -1.3 (95% CI -1.7 to -0.8, P < 0.001) and - 1.8 (95% CI -2.4 to -1.3, P < 0.001) in the control group, and -1.8 (95% CI -2.3 to - 1.4, P < 0.001) and -2.5 (95% CI -3.1 to -2.0, P < 0.001) in the Tα1 group. The decreasing tendency within 7 days in SOFA score seemed to favor the Tα1 group. The ratio of CD4+/CD8+ remained unchanged during the 7 days in both groups.

**Table 5 Dynamic changes of SOFA and laboratory measurements**

| **Measures** | **Control Group** | **Tα1 group** | **Between groups difference** |
|---|---|---|---|
| | Mean (95% CI) | Mean (95% CI) | |
| SOFA score | | | |
| Day 0 | 7.7 (6.8-8.5) | 7.9 (7.0-8.7) | |
| Day 3 | 6.4 (5.6-7.2) | 6.1 (5.2-6.9) | |
| Day 7 | 5.9 (5.0-6.7) | 5.3 (4.5-6.2) | |
| ΔDay 3* | -1.3 (-1.7-0.8)^{a} | -1.8 (-2.3-1.4)^{a} | -0.5 (-1.2-0.1) |
| ΔDay 7* | -1.8 (-2.4-1.3)^{a} | -2.5 (-3.1-2.0)^{a} | -0.7 (-1.5-0) |

| mHLA-DR (%) | | | |
|---|---|---|---|
| Day 0 | 58.2 (38.8-77.6) | 51.8 (32.5-71.2) | |
| Day 3 | 62.2 (42.8-81.6) | 59.8 (40.4-79.2) | |
| Day 7 | 69.4 (50.0-88.8) | 68.9 (49.5-88.2) | |
| ΔDay 3* | 4.1 (1.4-6.7)^{b} | 8.0 (5.4-10.5)^{b} | 3.9 (0.2-7.6)^{a} |
| ΔDay 7* | 11.2 (7.8-14.7)^{b} | 17.0 (13.7-20.3)^{b} | 5.8 (1.0-10.5)^{a} |

| **Measures** | **Control Group** | **Tα1 group** | **Between groups difference** |
|---|---|---|---|
| CD⁴⁺/DC⁸⁺ | | | |
| Day 0 | 2.4 (2.0-2.9) | 2.5 (2.0-2.9) | |
| Day 3 | 2.7 (2.2-3.1) | 2.7 (2.3-3.2) | |
| Day 7 | 2.4 (2.0-2.9) | 2.5 (2.1-3.0) | |
| ΔDay 3* | 0.2 (0-0.5) | 0.3 (0-0.5)^{a} | 0 (-0.3-0.4) |
| ΔDay 7* | 0 (-0.3-0.3) | 0.1 (-0.2-0.4) | 0.1 (-0.3-0.5) |

| | | | |
|---|---|---|---|
| * ΔDay 3 and ΔDay 7 were defined as the value changes on day 3 and day 7 compared with that on day 0. ^{a}*P* < 0.05; ^{b}*P* < 0.01. CD, cluster of differentiation; CI, confidence interval; mHLA-DR, monocyte human leukocyte antigen-DR; SOFA, Sequential Organ Failure Assessment; Tα1, thymosin alpha 1. | | | |

### Subgroup analysis

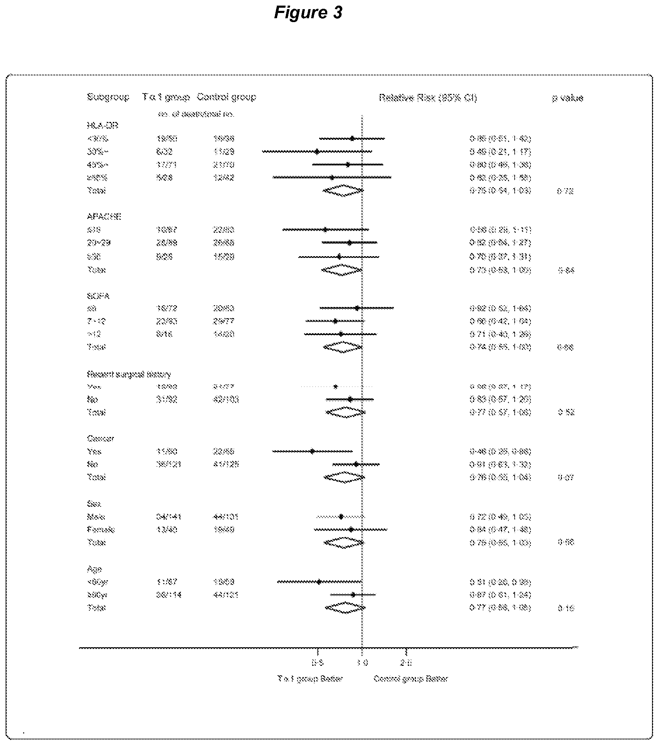

Mortality rates among prespecified subgroups of patients are shown in Figure 3. Prespecified analyses of the primary end point, where patients were stratified according to APACHE II score, SOFA score, mHLA-DR level, history of surgery or cancer, sex and age, showed that Tα1 tended to improve outcome but without statistical significance. In a subgroup analysis of patients with cancer, the relative risk of death of the Tα1 group when compared to the control group was 0.46 (95% CI 0.25 to 0.86, P = 0.01); on the other hand, in non-cancerous patients, the relative risk of death of the Tα1 group was 0.91 (P = 0.07 by the test of interaction).

### Adverse events

Safety and tolerability assessment of Tα1 (see Additional file 3) was based on the comparison of all available information obtained from the two groups with respect to detected outliers in laboratory safety data, drug-related serious adverse events (assessed by the investigator) and deterioration of organ and system function (assessed by the individual SOFA component scoring for respiratory, cardiovascular, hepatic, coagulation, renal and nervous systems that arose during the treatment).

In this study, no Tα1-related severe adverse event (SAE) was reported and no treatment was discontinued due to intolerance or adverse events. There were no statistically significant differences between the control and Tα1 group with regard to the frequency of outlying laboratory values and all-cause organ or system impairment (refer to Table 6).

**Table 6: Frequency of patients with outlying values of laboratory safety assays and all-cause organ and system impairment.**

| | **Control group (*n* = 180)** | **TαI group (*n* = 181)** | ***P* value** |
|---|---|---|---|
| **Laboratory safety assays** | | | |
| ALT (U/L) | 43 (23.9) | 38 (21.0) | 0.51 |
| AST (U/L) | 44 (24.4) | 43 (23.8) | 0.88 |
| Hypoglycemia | 9 (5.0) | 8 (4.4) | 0.79 |
| Hemoglobin (g/L) | 23 (12.8) | 27 (14.9) | 0.56 |
| Platelets 10³/mm³) | 77 (42.8) | 67 (37.0) | 0.26 |
| Creatinine (mmol/L) | 12 (6.7) | 18 (9.9) | 0.26 |

| **SOFA component scores*** | | | |
|---|---|---|---|
| Respiratory system | 27 (15.0) | 24 (13.3) | 0.64 |
| Coagulation system | 52 (28.9) | 48 (26.5) | 0.62 |
| Cardiovascular system | 21 (11.7) | 28 (15.5) | 0.29 |
| Hepatic system | 25 (13.9) | 21 (11.6) | 0.51 |
| Nervous system | 22 (12.2) | 14 (7.7) | 0.15 |
| Renal system | 19 (10.6) | 26 (14.4) | 0.27 |

| | | | |
|---|---|---|---|
| *Organ and system impairment based on the deterioration of SOFA component scores during the treatment. ALT, alanine aminotransferase; AST, aspartate aminotransferase; SOFA, Sequential Organ Failure Assessment; Tα1, thymosin alpha 1. | | | |

### DISCUSSION

Immune system dysregulation plays a significant role in the course of sepsis. Previously, it was believed that the exaggerated pro-inflammatory response and its associated inflammation-induced organ injury were the major factors leading to deaths in sepsis. However, recent studies indicate that heterogeneity exists in septic patients' immune response, with some appearing immunostimulated, whereas in others appearing sup-pressed [23]. Although both pro-inflammatory and anti-inflammatory drugs have been evaluated, few have yet been found to significantly reduce the mortality [24-26]. Tα1 is thought to have immunomodulating effects primarily affecting the augmentation of T-cell function [27,28]. Tα1 has also shown actions beyond its effect on T lymphocytes by acting as an endogenous regulator of both the innate and adaptive immune systems [11,29]. Tα1 plays a unique role in balancing pro- and anti-inflammatory cytokine production through the involvement of distinct Toll-like receptors (TLRs) acting on different dendritic cells (DC) subsets and involving the MyD88-dependent signaling pathway. Tα1 can increase IL-12, IL-2, IFN-α and IFN-γ secretion to present antimicrobial effect and increase IL-10 and percentage of regulatory T cells (Tregs) to control inflammation [11,30-32]. Therefore, is an appropriate immunoregulator for treating severe sepsis that is characterized by the large heterogeneity in immune function.

Our data suggested that the administration of Tα1 reduced 28-day mortality from any cause in patients with clinically diagnosed severe sepsis by 9.0%, with a marginal P value (P = 0.062 in the nonstratified analysis; log rank, P = 0.049) and decreased in-hospital mortality (P = 0.032). Our study was prospectively set up to detect an absolute 15% mortality reduction from an expected 50% as indicated in our previous trial and another epidemiology research about severe sepsis in China [22,33]. Mortality and drug effect size were not consistent with our expectation, which might lead to the marginal P value in the comparison of 28-day survival rate between two groups. In contrast to our results, previous trials in adults indicated that Tα1 significantly reduced mortality by 13.1% to 18% as compared to the control group [14-16]. The following reasons may explain this discrepancy in different trials. First, heterogeneity in patient populations and different therapeutic approaches could have influenced the outcomes; second, previous trials did not report the allocation concealment, which could have an unexpected impact on results. Schulz et al. indicated that the odds ratios were exaggerated by 41% for inadequately concealed trials and by 30% for unclearly concealed trials [34]. Third, those studies used more than one drug as therapeutic intervention and made it difficult to attribute the beneficial effects observed to each agent.

The most frequently assessed biomarker for evaluating immune function of severe sepsis is mHLA-DR. There seems to be a general consensus that diminished mHLA-DR is a reliable marker for the development of immunodysfuction in severe sepsis patients [35,36]. Recent studies indicate that the dynamic change of mHLA-DR over time was a better predictor of mortality and mHLA-DR recovery was associated with a better prognosis [21,37,38]. In the present trial, a greater improvement of mHLA-DR was observed in the Ta 1 group on day 3 and day 7 than in the control group, which suggests that Tα1 may improve immune function in severe sepsis. The ratio of CD4+/CD8+ is another parameter to evaluate immunological status in sepsis. Decreased CD4+/CD8+ ratio was related to the development of severe sepsis and multiple organ failure (MOF) in trauma patients [39]. Some studies showed that thymosin alpha 1 can increase CD4+/CD8+ ratio [40,41]. On the other side, one research study has indicated that mHLA-DR, not CD4+, CD8+ or ratio of CD4+/CD8+, can predict the prognosis of severe sepsis [42]. In our research, we did not find statistically significant difference in the CD4+/CD8+ ratio between the two groups. The decreasing tendency within 7 days in SOFA score seemed to favor the Tα1 group but with no significant difference in changes between the two groups. However, considering the fact that we observed the changes of these indices for only 7 days, there could have been some difference between the two groups if the observation had been extended to 14 or 28 days.

The median time from the first organ dysfunction detected to enrollment was more than 24 hrs in both groups, but longer in the Tα1 group. We adopted a retrospective method to determine the time window between the onset of the first organ dysfunction detected and study enrollment according to objective data (such as blood gas analysis), many of which were obtained before transferring the severe sepsis patients to the ICU [7]. However, those patients without indicative objective data could also have suffered from severe sepsis and the delay in laboratory tests could substantially underestimate the time after onset. In other words, the time after onset determined by laboratory tests in non-ICU departments was out of our control and subject to errors, especially when the estimation was based on hours instead of days. The precise time window between onset of the first organ dysfunction and enrollment could exceed the recorded time and could possibly be balanced between the two groups. The better way of enrolling severe sepsis patients in immunotherapy research may be through mHLA-DR value, which has been proved to be a good predictor to evaluate patients' immune status and a good parameter for individualized goal-directed therapy [43].

Reductions in the relative risk of death were observed in all subgroups including those stratified according to age, sex, APACHE II score, SOFA score and levels of mHLA-DR, but without statistical significance. The aim of analyzing different prespecified subgroups in our research was to prepare for our future research in targeted specific groups of severe sepsis patients who might benefit from the Tα1 treatment. The results of subgroup analysis in our research were inconclusive and whether Tα1 is more effective in specific groups of patients with severe sepsis and this should be explored in trials with a larger sample size.

Types of pathogen and empirical antibiotic therapy are very important factors that affect the outcome of severe sepsis. It is noted that the origins of microorganisms are substantially diverse in different areas and even in different hospitals in the same area. So is empirical therapy. In the present study, there was a high isolation rate of gram-negative bacteria (pseudomonas, acinetobacter) compared with some other epidemiology study of infection in ICU [44]. In fact, the relatively higher incidence of pseudomonas and acinetobacter infections is not unusual in China [33] so that the adequate empirical therapy is adjusted accordingly.

Thymosin alpha 1 has been shown to be a safe and well-tolerated agent in other studies [12,13]. Serious adverse events were not observed in our trial. Outlying laboratory values and all-cause organ and system impairment were similar in both groups. However, subjective sensations such as irritation or burning, general or gastrointestinal disorders were difficult to assess due to the severity of disease, sedation or analgesia in severe sepsis patients.

In our study, several factors limit the extent to which the results can be generalized. First, the study population was heterogeneous with respect to clinical features. Although over 80 baseline characteristics were comparable between the two groups, difference in mHLA-DR expression was present and was probably due to the heterogeneity in patients and the relatively small size of samples. In fact, unbalanced baseline characters between groups were not rare in severe sepsis trials even with large samples [45,46]. In our study, to assess whether outcomes differed by treatment groups, linear mixed models for longitudinal data were fit with adjustment for the baseline value. This method has been widely used in multicenter research [47,48]. Second, considering the heterogeneity of severe sepsis, some patient groups could benefit more from the intervention than other septic patients. The future individualized and goal-directed Tα1 treatment of severe sepsis should be implemented in targeted specific groups of patients. One of the biomarkers that can be used to stratify patients according to their immune status is mHLA-DR. Meisel *et al.* reported that mHLA-DR level was associated with immunosuppression status in sepsis patients who benefited from the granulocyte-macrophage colony-stimulating factor (GM-CSF) treatment [43]. We will try to adopt mHLA-DR target immunosuppression patients in future study. Third, since a considerable proportion of patients were transferred out of ICU within one week, which makes it difficult to guarantee that the complete laboratory and follow-up data could be obtained, we only collected laboratory data within 7 days and followed up the survival status for 28 days. A more extensive laboratory data collection and extended follow-up period could possibly provide more significant information. Fourth, there are few biomarkers to evaluate the immunological derangement. In the present trial, we adopted the widely used mHLA-DR. Fifth, from our trial the extension of the treatment to more than 7 days or the increase of dose could possibly generate a significant improvement in the outcomes of severe sepsis patients. Sixth, we did not adopt the double-blind method because no identical-appearing placebo was available and only the patients and the statistician were blinded. To minimize the potential bias, randomization and adequate allocation concealment were meticulous in the trial [34] and the primary and second end points were objective rather than subjective.

Given these limitations, the present research is a preliminary exploration on the efficacy of thymosin alpha 1 in severe sepsis and further double-blinded studies are needed.

### Conclusions

This RCT demonstrates that thymosin alpha 1 therapy in combination with conventional medical therapy may be effective in improving clinical outcomes in a targeted population of severe sepsis. Larger multicenter studies are indicated to confirm these findings.

In light of the crucial role of immunologic derangement in severe sepsis, immunotherapy may be an important adjunctive treatment.

This study demonstrates that immunodulation with thymosin alpha 1 may effectively improve outcomes of patients with severe sepsis. A beneficial impact on the immunofunction of patients with severe sepsis was also observed. Further researches are needed to confirm these findings.

### References

1. Heron M, Hoyert DL, Murphy SL, Xu J, Kochanek KD, Tejada-Vera B: Deaths: final data for 2006. Natl Vital Stat Rep 2009, 57:1-134.
2. Martin CM, Priestap F, Fisher H, Fowler RA, Heyland DK, Keenan SP, Longo CJ, Morrison T, Bentley D, Antman N, STAR Registry Investigators: A prospective, observational registry of patients with severe sepsis: the Canadian Sepsis Treatment and Response Registry. Crit Care Med 2009, 37:81-88.
3. Angus DC, Linde-Zwirble WT, Lidicker J, Clermont G, Carcillo J, Pinsky MR: Epidemiology of severe sepsis in the United States: analysis of incidence, outcome, and associated costs of care. Crit Care Med 2001, 29:1303-1310.
4. Levy MM, Dellinger RP, Townsend SR, Linde-Zwirble WT, Marshall JC, Bion J, Schorr C, Artigas A, Ramsay G, Beale R, Parker MM, Gerlach H, Reinhart K, Silva E, Harvey M, Regan S, Angus DC: The Surviving Sepsis Campaign: results of an international guideline-based performance improvement program targeting severe sepsis. Intensive Care Med 2010, 36:222-231.
5. Finfer S, Bellomo R, Lipman J, French C, Dobb G, Myburgh J: Adult-population incidence of severe sepsis in Australian and New Zealand intensive care units. Intensive Care Med 2004, 30:589-596.
6. Brun-Buisson C, Meshaka P, Pinton P, Vallet B, EPISEPSIS Study Group: EPISEPSIS: a reappraisal of the epidemiology and outcome of severe sepsis in French intensive care units. Intensive Care Med 2004, 30:580-588.
7. Bernard GR, Vincent JL, Laterre PF, LaRosa SP, Dhainaut JF, Lopez-Rodriguez A, Steingrub JS, Garber GE, Helterbrand JD, Ely EW, Fisher CJ Jr, Recombinant human protein C Worldwide Evaluation in Severe Sepsis (PROWESS) study group: Efficacy and safety of recombinant human activated protein C for severe sepsis. N Engl J Med 2001, 344:699-709.
8. Hotchkiss RS, Karl IE: The pathophysiology and treatment of sepsis. N Engl J Med 2003, 348:138-150.
9. Hotchkiss RS, Opal S: Immunotherapy for sepsis - a new approach against an ancient foe. N Engl J Med 2010, 363:87-89.
10. Goldstein AL, Guha A, Zatz MM, Hardy MA, White A: Purification and biological activity of thymosin, a hormone of the thymus gland. Proc Natl Acad Sci USA 1972, 69:1800-1803.
11. Romani L, Bistoni F, Montagnoli C, Gaziano R, Bozza S, Bonifazi P, Zelante T, Moretti S, Rasi G, Garaci E, Puccetti P: Thymosin alpha1: an endogenous regulator of inflammation, immunity, and tolerance. Ann N Y Acad Sci 2007, 1112:326-338.
12. Goldstein AL: From lab to bedside: emerging clinical applications of thymosin alpha 1. Expert Opin Biol Ther 2009, 9:593-608.
13. Tuthill C, Rios I, McBeath R: Thymosin alpha 1: past clinical experience and future promise. Ann N Y Acad Sci 2010, 1194:130-135.
14. Chen H, He MY, Li YM: Treatment of patients with severe sepsis using ulinastatin and thymosin alpha1: a prospective, randomized, controlled pilot study. Chin Med J (Engl) 2009,122:883-888.
15. Li Y, Chen H, Li X, Zhou W, He M, Chiriva-Internati M, Wachtel MS, Frezza EE: A new immunomodulatory therapy for severe sepsis: Ulinastatin Plus Thymosin {alpha} 1. J Intensive Care Med 2009, 24:47-53.
16. Lin HY: [Clinical trial with a new immunomodulatory strategy: treatment of severe sepsis with Ulinastatin and Maipuxin]. Zhonghua Yi Xue Za Zhi 2007, 87:451-457.
17. Dellinger RP, Levy MM, Carlet JM, Bion J, Parker MM, Jaeschke R, Reinhart K, Angus DC, Brun-Buisson C, Beale R, Calandra T, Dhainaut JF, Gerlach H, Harvey M, Marini JJ, Marshall J, Ranieri M, Ramsay G, Sevransky J, Thompson BT, Townsend S, Vender JS, Zimmerman JL, Vincent JL, International Surviving Sepsis Campaign Guidelines Committee; American Association of Critical-Care Nurses; American College of Chest Physicians; American College of Emergency Physicians; Canadian Critical Care Society; European Society of Clinical Microbiology and Infectious Diseases, et al: Surviving Sepsis Campaign: international guidelines for management of severe sepsis and septic shock: 2008. Intensive Care Med 2008, 34:17-60.
18. Guidelines for the management of adults with hospital-acquired, ventilator-associated, and healthcare-associated pneumonia. Am J Respir Crit Care Med 2005, 171:388-416.
19. Degoricija V, Sharma M, Legac A, Gradiser M, Sefer S, Vucicevic Z: Survival analysis of 314 episodes of sepsis in medical intensive care unit in university hospital: impact of intensive care unit performance and antimicrobial therapy. Croat Med J 2006, 47:385-397.
20. Garnacho-Montero J, Garcia-Garmendia JL, Barrero-Almodovar A, Jimenez-Jimenez FJ, Perez-Paredes C, Ortiz-Leyba C: Impact of adequate empirical antibiotic therapy on the outcome of patients admitted to the intensive care unit with sepsis. Crit Care Med 2003, 31:2742-2751.
21. Wu JF, Ma J, Chen J, Ou-Yang B, Chen MY, Li LF, Liu YJ, Lin AH, Guan XD: Changes of monocyte human leukocyte antigen-DR expression as a reliable predictor of mortality in severe sepsis. Crit Care 2011, 15:R220.
22. Huang SW, Guan XD, Chen J, OuYang B: [Clinical study and long-term evaluation of immunomodulation therapy on trauma, severe sepsis and multiple organ dysfunction syndrome patients]. Zhongguo Wei Zhong Bing Ji Jiu Yi Xue 2006, 18:653-656.
23. Remick DG: Pathophysiology of sepsis. Am J Pathol 2007, 170:1435-1444.
24. Abraham E, Wunderink R, Silverman H, Perl TM, Nasraway S, Levy H, Bone R, Wenzel RP, Balk R, Pennington JE, Wherry JC, TNF-alpha MAb Sepsis Study Group: Efficacy and safety of monoclonal antibody to human tumor necrosis factor alpha in patients with sepsis syndrome. A randomized, controlled, double-blind, multicenter clinical trial. JAMA 1995, 273:934-941.
25. Panacek EA, Marshall JC, Albertson TE, Johnson DH, Johnson S, MacArthur RD, Miller M, Barchuk WT, Fischkoff S, Kaul M, Teoh L, Van Meter L, Daum L, Lemeshow S, Hicklin G, Doig C, Monoclonal Anti-TNF: a Randomized Controlled Sepsis Study Investigators: Efficacy and safety of the monoclonal antitumor necrosis factor antibody F(ab')2 fragment afelimomab in patients with severe sepsis and elevated interleukin-6 levels. Crit Care Med 2004, 32:2173-2182.
26. Docke WD, Randow F, Syrbe U, Krausch D, Asadullah K, Reinke P, Volk HD, Kox W: Monocyte deactivation in septic patients: restoration by IFN-gamma treatment. Nat Med 1997, 3:678-681.
27. Serrate SA, Schulof RS, Leondaridis L, Goldstein AL, Sztein MB: Modulation of human natural killer cell cytotoxic activity, lymphokine production, and interleukin 2 receptor expression by thymic hormones. J Immunol 1987, 139:2338-2343.
28. Sztein MB, Serrate SA: Characterization of the immunoregulatory properties of thymosin alpha 1 on interleukin-2 production and interleukin-2 receptor expression in normal human lymphocytes. Int J Immunopharmacol 1989,11:789-800.
29. Pierluigi B, D'Angelo C, Fallarino F, Moretti S, Zelante T, Bozza S, De Luca A, Bistoni F, Garaci E, Romani L: Thymosin alpha1: the regulator of regulators? Ann N Y Acad Sci 2010, 1194:1-5.
30. Romani L, Bistoni F, Gaziano R, Bozza S, Montagnoli C, Perruccio K, Pitzurra L, Bellocchio S, Velardi A, Rasi G, Di Francesco P, Garaci E: Thymosin alpha 1 activates dendritic cells for antifungal Th1 resistance through toll-like receptor signaling. Blood 2004, 103:4232-4239.
31. Bozza S, Gaziano R, Bonifazi P, Zelante T, Pitzurra L, Montagnoli C, Moretti S, Castronari R, Sinibaldi P, Rasi G, Garaci E, Bistoni F, Romani L: Thymosin alpha1 activates the TLR9/MyD88/IRF7-dependent murine cytomegalovirus sensing for induction of anti-viral responses in vivo. Int Immunol 2007, 19:1261-1270.
32. Yang X, Qian F, He HY, Liu KJ, Lan YZ, Ni B, Tian Y, Fu XL, Zhang J, Shen ZG, Li J, Yin Y, Li JT, Wu YZ: Effect of thymosin alpha-1 on subpopulations of Th1, Th2, Th17, and regulatory T cells (Tregs) in vitro. Braz J Med Biol Res 2012, 45:25-32.
33. Cheng B, Xie G, Yao S, Wu X, Guo Q, Gu M, Fang Q, Xu Q, Wang D, Jin Y, Yuan S, Wang J, Du Z, Sun Y, Fang X: Epidemiology of severe sepsis in critically ill surgical patients in ten university hospitals in China. Crit Care Med 2007, 35:2538-2546.
34. Schulz KF, Grimes DA: Allocation concealment in randomised trials: defending against deciphering. Lancet 2002, 359:614-618.
35. Monneret G, Venet F, Pachot A, Lepape A: Monitoring immune dysfunctions in the septic patient: a new skin for the old ceremony. Mol Med 2008,14:64-78.
36. Schefold JC: Measurement of monocytic HLA-DR (mHLA-DR) expression in patients with severe sepsis and septic shock: assessment of immune organ failure. Intensive Care Med 2010, 36:1810-1812.
37. Lukaszewicz AC GM, Resche-Rigon M, Pirracchio R, Faivre V, Boval B, Payen D: Monocytic HLA-DR expression in intensive care patients: interest for prognosis and secondary infection prediction. Crit Care Med 2009, 37:2746-2752.
38. Monneret G, Lepape A, Voirin N, Bohe J, Venet F, Debard AL, Thizy H, Bienvenu J, Gueyffier F, Vanhems P: Persisting low monocyte human leukocyte antigen-DR expression predicts mortality in septic shock. Intensive Care Med 2006, 32:1175-1183.
39. Menges T, Engel J, Welters I, Wagner RM, Little S, Ruwoldt R, Wollbrueck M, Hempelmann G: Changes in blood lymphocyte populations after multiple trauma: association with posttraumatic complications. Crit Care Med 1999, 27:733-740.
40. Zhang Y, Chen H, Li YM, Zheng SS, Chen YG, Li LJ, Zhou L, Xie HY, Praseedom RK: Thymosin alpha1 - and ulinastatin-based immunomodulatory strategy for sepsis arising from intra-abdominal infection due to carbapenem-resistant bacteria. J Infect Dis 2008, 198:723-730.
41. Wang X, Li W, Niu C, Pan L, Li N, Li J: Thymosin alpha 1 is associated with improved cellular immunity and reduced infection rate in severe acute pancreatitis patients in a double-blind randomized control study. Inflammation 2011, 34:198-202.
42. Saenz JJ, Izura JJ, Manrique A, Sala F, Gaminde I: Early prognosis in severe sepsis via analyzing the monocyte immunophenotype. Intensive Care Med 2001, 27:970-977.
43. Meisel C, Schefold JC, Pschowski R, Baumann T, Hetzger K, Gregor J, Weber-Carstens S, Hasper D, Keh D, Zuckermann H, Reinke P, Volk HD: Granulocyte-macrophage colony-stimulating factor to reverse sepsis-associated immunosuppression: a double-blind, randomized, placebo-controlled multicenter trial. Am J Respir Crit Care Med 2009, 180:640-648.
44. Alberti C, Brun-Buisson C, Burchardi H, Martin C, Goodman S, Artigas A, Sicignano A, Palazzo M, Moreno R, Boulme R, Lepage E, Le Gall R: Epidemiology of sepsis and infection in ICU patients from an international multicentre cohort study. Intensive Care Med 2002, 28:108-121.
45. Ranieri VM, Thompson BT, Barie PS, Dhainaut JF, Douglas IS, Finfer S, Gardlund B, Marshall JC, Rhodes A, Artigas A, Payen D, Tenhunen J, Al-Khalidi HR, Thompson V, Janes J, Macias WL, Vangerow B, Williams MD, PROWESS-SHOCK Study Group: Drotrecogin alfa (activated) in adults with septic shock. N Engl J Med 2012, 366:2055-2064.
46. Brunkhorst FM, Engel C, Bloos F, Meier-Hellmann A, Ragaller M, Weiler N, Moerer O, Gruendling M, Oppert M, Grond S, Olthoff D, Jaschinski U, John S, Rossaint R, Welte T, Schaefer M, Kern P, Kuhnt E, KiehntopfM, Hartog C, Natanson C, Loeffler M, Reinhart K, German Competence Network Sepsis (SepNet): Intensive insulin therapy and pentastarch resuscitation in severe sepsis. N Engl J Med 2008, 358:125-139.
47. DeFronzo RA, Tripathy D, Schwenke DC, Banerji M, Bray GA, Buchanan TA, Clement SC, Henry RR, Hodis HN, Kitabchi AE, Mack WJ, Mudaliar S, Ratner RE, Williams K, Stentz FB, Musi N, Reaven PD, ACT NOW Study: Pioglitazone for diabetes prevention in impaired glucose tolerance. N Engl J Med 2011, 364:1104-1115.
48. TODAY Study Group, Zeitler P, Hirst K, Pyle L, Linder B, Copeland K, Arslanian S, Cuttler L, Nathan DM, Tollefsen S, Wilfley D, Kaufman F: A clinical trial to maintain glycemic control in youth with type 2 diabetes. N Engl J Med 2012, 366:2247-2256.

## Claims

1. Alpha thymosin peptide for use in a method for treating sepsis in a human, the method comprising administering a regimen of alpha thymosin peptide to the human, optionally the sepsis is due to bacterial, fungal or viral infection, wherein the administration does not contain a protease inhibitor, and wherein the treatment reduces mortality.

2. Alpha thymosin peptide for use according to claim 1, wherein the human is immune deficient.

3. Alpha thymosin peptide for use according to claim 1, wherein the sepsis is hospital acquired.

4. Alpha thymosin peptide for use according to claim 1, wherein in the method the alpha thymosin peptide is administered at a dose of at least about 0.5 mg per day, optionally the alpha thymosin peptide is administered at about 1 .6 to about 6.4 mg per day or about 1 .6 mg or 3.2 mg per day.

5. Alpha thymosin peptide for use according to claim 1, wherein in the method the alpha thymosin peptide is administered by intravenous or subcutaneous injection, optionally the alpha thymosin peptide is administered by continuous infusion.

6. Alpha thymosin peptide for use according to claim 1, wherein the regimen involves administering the alpha thymosin peptide from 1 to 4 times daily, optionally the alpha thymosin administrations are given approximately twice per day or once per day.

7. Alpha thymosin peptide for use according to claim 1, wherein in the method the alpha thymosin peptide is administered twice per day for at least 5 days, optionally the alpha thymosin peptide is administered twice per day for about 5 to 14 days.

8. Alpha thymosin peptide for use according to claim 1, wherein in the method the alpha thymosin peptide is administered twice per day for at least 5 days followed by once per day for at least two days, optionally for about 2 to 7 days.

9. Alpha thymosin peptide for use according to claim 1, wherein the human shows one or more signs or symptoms of an infection.

10. Alpha thymosin peptide for use according to claim 1, wherein the human shows one or more signs or symptoms of sepsis.

11. Alpha thymosin peptide for use according to claim 1, wherein in the method the alpha thymosin peptide is administered within at least the first 24 hours, 48 hours, 72 hours, or 96 hours of showing one or more signs or symptoms of an infection or sepsis.

12. Alpha thymosin peptide for use according to claim 1, wherein the sepsis is confirmed by a diagnostic test.

13. Alpha thymosin peptide for use according to claim 1, wherein in the method the regimen of alpha thymosin peptide is administered concurrently with antibacterial, antiviral, or antifungal therapy.

14. Alpha thymosin peptide for use according to claim 1, wherein the sepsis is associated with an infectious microorganism selected from the group consisting of *Lysteria monocytogenes, Pseudomonas sp.* (e.g., *P. aeruginosa), Serratia marcescens, Clostridium difficile, Staphylococcus aureus, Staphylococcus sp., Acinetobacter spp*., *Enterococcus sp., Enterobacter sp., E. coli, Klebsiella sp., Streptococcus* (e.g., *S. pneumoniae), Haemophilus influenzae* and *Neisseria meningitidis,* optionally the sepsis is associated with a drug resistant or multi-drug resistant *Staphylococcus aureus, Staphylococcus sp., Enterococcus sp., Pseudomonas sp., Klebsiella sp., E. coli,* or *Clostridium Difficile.*

15. Alpha thymosin peptide for use according to claim 1, wherein the sepsis is associated with methicillin-resistant or vancomycin-resistant *Staphylococcus aureus.*

## Patentansprüche

1. Alpha-Thymosinpeptid zur Verwendung in einem Verfahren zur Behandlung von Sepsis bei einem Menschen, wobei das Verfahren das Verabreichen eines Alpha-Thymosinpeptid-Therapieschemas an den Menschen beinhaltet, wobei die Sepsis optional durch eine bakterielle, Pilz- oder Virusinfektion bedingt ist, wobei die Verabreichung keinen Proteasehemmer enthält und wobei die Behandlung die Mortalität reduziert.

2. Alpha-Thymosinpeptid zur Verwendung gemäß Anspruch 1, wobei der Mensch immungeschwächt ist.

3. Alpha-Thymosinpeptid zur Verwendung gemäß Anspruch 1, wobei die Sepsis nosokomial ist.

4. Alpha-Thymosinpeptid zur Verwendung gemäß Anspruch 1, wobei in dem Verfahren das Alpha-Thymosinpeptid in einer Dosis von mindestens etwa 0,5 mg täglich verabreicht wird, wobei das Alpha-Thymosinpeptid optional in einer Dosis von etwa 1,6 bis etwa 6,4 mg täglich oder etwa 1,6 mg oder 3,2 mg täglich verabreicht wird.

5. Alpha-Thymosinpeptid zur Verwendung gemäß Anspruch 1, wobei in dem Verfahren das Alpha-Thymosinpeptid durch intravenöse oder subkutane Injektion verabreicht wird, wobei das Alpha-Thymosinpeptid optional durch Dauerinfusion verabreicht wird.

6. Alpha-Thymosinpeptid zur Verwendung gemäß Anspruch 1, wobei das Therapieschema das Verabreichen des Alpha-Thymosinpeptids von 1 bis 4 Mal täglich umfasst, wobei die Alpha-Thymosinverabreichungen optional ungefähr zweimal täglich oder einmal täglich gegeben werden.

7. Alpha-Thymosinpeptid zur Verwendung gemäß Anspruch 1, wobei in dem Verfahren das Alpha-Thymosinpeptid mindestens 5 Tage lang zweimal täglich verabreicht wird, wobei das Alpha-Thymosinpeptid optional etwa 5 bis 14 Tage lang zweimal täglich verabreicht wird.

8. Alpha-Thymosinpeptid zur Verwendung gemäß Anspruch 1, wobei in dem Verfahren das Alpha-Thymosinpeptid mindestens 5 Tage lang zweimal täglich und danach mindestens 2 Tage lang einmal täglich, optional etwa 2 bis 7 Tage lang verabreicht wird.

9. Alpha-Thymosinpeptid zur Verwendung gemäß Anspruch 1, wobei bei dem Menschen ein oder mehrere Zeichen oder Symptome einer Infektion aufgetreten sind.

10. Alpha-Thymosinpeptid zur Verwendung gemäß Anspruch 1, wobei bei dem Menschen ein oder mehrere Zeichen oder Symptome einer Sepsis aufgetreten sind.

11. Alpha-Thymosinpeptid zur Verwendung gemäß Anspruch 1, wobei in dem Verfahren das Alpha-Thymosinpeptid innerhalb mindestens der ersten 24 Stunden, 48 Stunden, 72 Stunden oder 96 Stunden nach dem Auftreten von einem oder mehreren Zeichen oder Symptomen einer Infektion oder Sepsis verabreicht wird.

12. Alpha-Thymosinpeptid zur Verwendung gemäß Anspruch 1, wobei die Sepsis durch einen diagnostischen Test bestätigt wird.

13. Alpha-Thymosinpeptid zur Verwendung gemäß Anspruch 1, wobei in dem Verfahren das Alpha-Thymosinpeptid-Therapieschema gleichzeitig mit antibakterieller, Antivirus- oder Antipilztherapie verabreicht wird.

14. Alpha-Thymosinpeptid zur Verwendung gemäß Anspruch 1, wobei die Sepsis mit einem infektiösen Mikroorganismus assoziiert ist, der aus der Gruppe ausgewählt ist, die aus Lysteria monocytogenes, Pseudomonas sp. (z. B. P. aeruginosa), Serratia marcescens, Clostridium difficile, Staphylococcus aureus, Staphylococcus sp., Acinetobacter spp., Enterococcus sp., Enterobacter sp., E. coli, Klebsiella sp., Streptococcus (z. B. S. pneumoniae), Haemophilus influenzae und Neisseria meningitidis besteht, wobei die Sepsis optional mit einem Arzneimittel-resistenten oder multiresistenten Staphylococcus aureus, Staphylococcus sp., Enterococcus sp., Pseudomonas sp., Klebsiella sp., E. coli oder Clostridium difficile assoziiert ist.

15. Alpha-Thymosinpeptid zur Verwendung gemäß Anspruch 1, wobei die Sepsis mit Methicillin-resistentem oder Vancomycin-resistentem Staphylococcus aureus assoziiert ist.

## Revendications

1. Peptide thymosine alpha pour une utilisation dans une méthode de traitement d'un sepsis chez un humain, la méthode comprenant l'administration d'un schéma thérapeutique du peptide thymosine alpha à l'humain, le sepsis étant éventuellement dû à une infection bactérienne, fongique ou virale, où l'administration ne contient pas un inhibiteur de protéase et où le traitement réduit la mortalité.

2. Peptide thymosine alpha pour une utilisation selon la revendication 1, où l'humain est immunodéficient.

3. Peptide thymosine alpha pour une utilisation selon la revendication 1, où le sepsis est un sepsis nosocomial.

4. Peptide thymosine alpha pour une utilisation selon la revendication 1 où, dans la méthode, le peptide thymosine alpha est administré à une dose d'environ 0,5 mg par jour au moins, éventuellement où le peptide thymosine alpha est administré à d'environ 1,6 à environ 6,4 mg par jour ou à environ 1,6 mg ou 3,2 mg par jour.

5. Peptide thymosine alpha pour une utilisation selon la revendication 1 où, dans la méthode, le peptide thymosine alpha est administré en injection intraveineuse ou sous-cutanée, éventuellement où le peptide thymosine alpha est administré en perfusion continue.

6. Peptide thymosine alpha pour une utilisation selon la revendication 1, où le schéma thérapeutique fait intervenir l'administration du peptide thymosine alpha de 1 à 4 fois par jour, éventuellement où les administrations de thymosine alpha sont effectuées approximativement deux fois par jour ou une fois par jour.

7. Peptide thymosine alpha pour une utilisation selon la revendication 1 où, dans la méthode, le peptide thymosine alpha est administré deux fois par jour pendant 5 jours au moins, éventuellement où le peptide thymosine alpha est administré deux fois par jour pendant environ 5 à 14 jours.

8. Peptide thymosine alpha pour une utilisation selon la revendication 1 où, dans la méthode, le peptide thymosine alpha est administré deux fois par jour pendant 5 jours au moins puis une fois par jour pendant deux jours au moins, éventuellement pendant environ 2 à 7 jours.

9. Peptide thymosine alpha pour une utilisation selon la revendication 1, où l'humain présente un ou plusieurs signes ou symptômes d'une infection.

10. Peptide thymosine alpha pour une utilisation selon la revendication 1, où l'humain présente un ou plusieurs signes ou symptômes d'un sepsis.

11. Peptide thymosine alpha pour une utilisation selon la revendication 1 où, dans la méthode, le peptide thymosine alpha est administré au moins dans les premières 24 heures, 48 heures, 72 heures ou 96 heures qui suivent le développement d'un ou plusieurs signes ou symptômes d'une infection ou d'un sepsis.

12. Peptide thymosine alpha pour une utilisation selon la revendication 1, où le sepsis est confirmé par un test diagnostique.

13. Peptide thymosine alpha pour une utilisation selon la revendication 1 où, dans la méthode, le schéma thérapeutique du peptide thymosine alpha est administré en conjonction avec une thérapie antibactérienne, antivirale ou antifongique.

14. Peptide thymosine alpha pour une utilisation selon la revendication 1, où le sepsis est associé à un micro-organisme infectieux sélectionné dans le groupe consistant en *Listeria monocytogenes, Pseudomonas sp.* (p. ex. *P. aeruginosa), Serratia marcescens, Clostridium difficile, Staphylococcus aureus, Staphylococcus sp., Acinetobacter spp., Enterococcus sp., Enterobacter sp., E. coli, Klebsiella sp., Streptococcus* (p. ex. *S. pneumoniae*), *Haemophilus influenzae* et *Neisseria meningitidis,* éventuellement où le sepsis est associé à une souche pharmacorésistante ou multipharmacorésistante de *Staphylococcus aureus,* de *Staphylococcus sp., d'Enterococcus sp.,* de *Pseudomonas sp.,* de *Klebsiella sp., d'E. coli* ou de *Clostridium difficile.*

15. Peptide thymosine alpha pour une utilisation selon la revendication 1, où le sepsis est associé à une souche de *Staphylococcus aureus* résistante à la méticilline ou résistante à la vancomycine.
